# EUROPEAN PATENT APPLICATION

(11) **EP 3 395 944 A1**
(43) Date of publication of application: **31.10.2018**
(21) Application number: 17168405.3
(22) Date of filing: 27.04.2017
(51) Int. Cl.: C12N 9/02, C12N 9/10, C12N 9/58

(54) **POLYNUCLEOTIDES ENCODING FOR POLYPEPTIDES INVOLVED IN THE OMPHATOLIN BIOSYNTHESIS**

(71) Applicant: Technische Universität Berlin, 10623 Berlin (DE)
(72) Inventor: RAMM, Sascha, 10179 Berlin (DE); MÜHLENWEG, Agnes, 13465 Berlin (DE); POCH-HASNEK, Annette, 13467 Berlin (DE); MÖSKER, Eva, 15366 Neuenhagen (DE); SÜßMUTH, Roderich, 10629 Berlin (DE); KRAWCZYK, Bartlomiej, 88400 Biberach an der Riss (DE)
(74) Representative: Maikowski & Ninnemann Patentanwälte Partnerschaft mbB

(57) **Abstract**

The present invention refers to polynucleotides encoding for polypeptides involved in the omphatolin biosynthesis, the corresponding polypeptides, a vector comprising said polynucleotiedes and host cells comprising said vector.

## Description

The present invention relates to polynucleotides encoding for polypeptides according to one the claims 1-6, polypeptides encoded by said polynucleotides according to claim 8, an expression vector according to claim 9, a host cell according to 11 and a process for obtaining at least one omphalotin compound according to claim 13.

### Description

Omphalotin A (Scheme 1) is a head-to-tail cyclized all-L-dodecapeptide produced by the basidiomycete *Omphalotus olearius.* It displays strong and selective activity against the plant pathogen nematode *Meloidogyne incognita* and shows no further phytotoxic, antibacterial or antifungal activities.

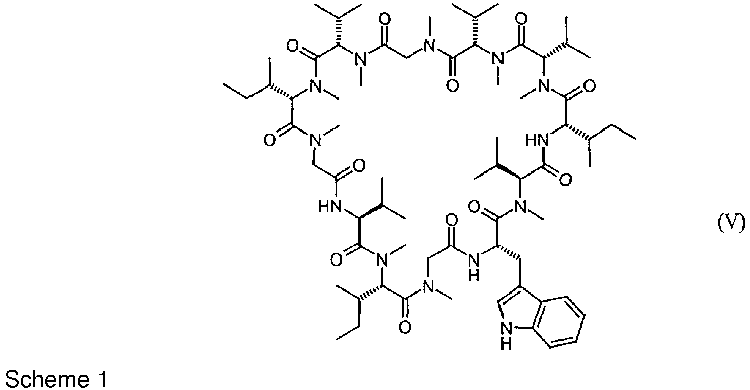

The challenging total synthesis has been accomplished by Jung and co-workers facilitating a profiling of its bioactivity (Angew. Chem. Int. Ed Engl. 2002, 41, 2307-2309).

In addition to the main compound omphalotin A, the derivatives omphalotin B to I, display various modifications e.g. acetylations, hydroxylations and an unusual tryptophan modification. A unifying feature of all omphalotins are nine *N*-methylations of the amide backbone. First isolated and described in 1997 the biosynthesis of the omphalotins was suspected as produced by non-ribosomal peptide synthetases (NRPS), as it has been described for cyclosporine, a structurally related *cyclo*-undecapeptide with 7 methylations and one D-amino acid (D-Ala).

Due to its high potent activity against nematodes, it would be desireable to provide an approach to obtain omphatolin and derivatives thereof in an easy manner.

The object of the present invention is therefore to provide a reliable biosynthetic approach for synthesizing omphatolin and derivatives thereof.

Accordingly, several polynucleotides encoding for polypeptides involved in the biosynthesis of omphatolin are provided.

Specifically, a first polynucleotide encoding for a polypeptide, in particular with N-methyltransferase activity and omphatolin precursor peptide activity, consisting essentially of Seq ID No. 1 or a polynucleotide with at least 70%, preferably at least 90%, more preferably at least 95 % identity thereto is provided. The polynucleotide of Seq ID No.1 comprises introns. The corresponding polynucleotide (cDNA) without introns is represented by Seq ID No. 2.

Seq. ID No 1 and Seq. ID No 2 comprise preferably polynucleotide *ophMA* of *Omphalotus olearius* encoding a polypeptide with N-methyltransferase activity and omphatolin precursor peptide activity. Thus, polynucleotide *ophMA* encodes for a fusion protein between a methlytransferase OphM and a precursor peptide OphA.

The 3'-region of the polynucleotides of Seq ID No. 1 and 2 comprises in each case a polynucleotide sequence encoding for a leader sequence (represented by Seq ID No. 3), a polynucleotide sequence encoding for a core sequence (represented by Seq ID No. 4) and a polynucleotide sequence encoding for a follower sequence (represented by Seq ID No.5). It is to be understood that any polynucleotide with at least 70%, preferably at least 90%, more preferably at least 95 % identity to Seq. ID No. 3-5 is also covered by the present invention.

The core sequence encodes for a polypeptide forming the backbone of the omphalotin compound. Specifically, the core sequence encodes for 12 amino acids that - after methylation - form the backbone of omphatolin A; either in cyclic form or also possibly in linear form.

Furthermore, another polynucleotide encoding for a polypeptide, in particular with prolyloligopeptidase activity, consisting essentially of Seq ID No. 6 or a polynucleotide with at least 70%, preferably at least 90%, more preferably at least 95 % identity thereto is provided. In particular, Seq ID No. 6 comprises preferably polynucleotide *ophP* of *Omphalotus olearius* encoding a polypetide with prolyloligopeptidase activity.

The polynucleotide of Seq ID No. 6 comprises introns. The corresponding polynucleotide (cDNA) without introns is represented by Seq ID No. 7.

A yet further polynucleotide encoding for a polypeptide, in particular with monooxygenase activity, consisting essentially of Seq ID No. 8 or a polynucleotide with at least 70%, preferably at least 90%, more preferably at least 95 % identity thereto is provided. In particular, Seq ID No. 3 comprises preferably polynucleotide *ophM1* of *Omphalotus olearius* encoding for a first monooxygenase activity. The polynucleotide of Seq ID No. 8 comprises introns.

Another polynucleotide encoding for a polypeptide, in particular with monooxygenase activity, consisting essentially of Seq ID No. 9 or a polynucleotide with at least 70%, preferably at least 90%, more preferably at least 95 % identity thereto is provided. In particular, Seq ID No. 9 comprises preferably polynucleotide *ophM2* of *Omphalotus olearius* encoding for a second monooxygenase activity. It is to be understood that Seq ID No 9 (or *ophM2*) comprises introns and Seq ID No. 10 *(ophM2a*) represents the cDNA of *ophM2.*

Furthermore, a another polynucleotide encoding for a polypeptide, in particular with acetyltransferase activity, consisting essentially of Seq ID No. 11 or a polynucleotide with at least 70%, preferably at least 90%, more preferably at least 95 % identity thereto is provided. In particular, Seq ID No. 11 comprises preferably polynucleotide *ophA* of *Omphalotus olearius* encoding for an acetyltransferase activity. It is to be understood that Seq ID No 11 (or *ophA*) comprises introns and Seq ID No. 12 *(ophAa*) represents the cDNA of *ophA..*

Finally, a polynucleotide encoding for polypeptides with activity for omphalotin synthesis consisting essentially of Seq ID No. 13 or a polynucleotide with at least 70%, preferably at least 90%, more preferably at least 95 % identity thereto is provided. The polynucleotide of Seq ID No. 13 comprises preferably at least one of the Seq ID No.1 - 12. In particular Seq ID No. 13 comprises *ophMA, ophP, ophM1, ophM2* and *ophA.*

It is to be understood that polynucleotides from other sources may be included into the present invention as long as the polynucleotides have at least an identity of 70% to claimed polynucleotides.

In one embodiment, said polynucleotides may be found in *Lentinula edodes* and may comprise a methylase according to Seq ID No. 20, a prolyl oligopeptidase according to Seq ID No. 21 and a cluster with biosynthesis activity according to Seq ID No. 22.

The "polynucleotides" or "nucleic acids" of the present invention may be in the form of RNA or in the form of DNA; DNA should be understood to include cDNA, genomic DNA, recombinant DNA and synthetic DNA. The DNA may be double-stranded or single-stranded and, if single stranded, may be the coding strand or non-coding (antisense) strand. The RNA may be mRNA.

The present invention also includes polynucleotides where the coding sequence for the polypeptide may be fused in the same reading frame to a polynucleotide sequence which aids in expression and secretion of a polypeptide from a host cell; for example, a leader sequence which functions as a secretory sequence for controlling transport of a polypeptide from the cell may be so fused. The polypeptide having such a leader sequence is termed a preprotein or a preproprotein and may have the leader sequence cleaved, by the host cell to form the mature form of the protein. These polynucleotides may have a 5' extended region so that it encodes a proprotein, which is the mature protein plus additional amino acid residues at the N-terminus. The expression product having such a prosequence is termed a proprotein, which is an inactive form of the mature protein; however, once the prosequence is cleaved an active mature protein remains. The additional sequence may also be attached to the protein and be part of the mature protein. Thus, for example, the polynucleotides of the present invention may encode polypeptides, or proteins having a prosequence, or proteins having both a prosequence and a presequence (leader sequence). The polynucleotides of the present invention may also have the coding sequence fused in frame to a marker sequence which allows for purification of the synthesized polypeptides.

The present invention is considered to further provide polynucleotides which hybridize to the hereinabove-described sequences wherein there is at least 70%, preferably at least 90%, and more preferably at least 95% identity or similarity between the sequences, and thus encode proteins having similar biological activity. Moreover, as known in the art, there is "similarity" between two polypeptides when the amino acid sequences contain the same or conserved amino acid substitutes for each individual residue in the sequence. Identity and similarity may be measured using sequence analysis software (e. g., ClustalW at PBIL (Pôle Bioinformatique Lyonnais) http://npsa-pbil.ibcp.fr). The present invention particularly provides such polynucleotides, which hybridize under stringent conditions to the hereinabove-described polynucleotides. As herein used, the term "stringent conditions" means conditions which permit hybridization between polynucleotides sequences and the polynucleotide sequences of SEQ ID No. 1-6 where there is at least about 70% identity.

Suitably stringent conditions can be defined by, e. g., the concentrations of salt or formamide in the prehybridization and hybridization solutions, or by the hybridization temperature, and are well known in the art. In particular, stringency can be increased by reducing the concentration of salt, by increasing the concentration of formamide, and/or by raising the hybridization temperature.

For example, hybridization under high stringency conditions may employ about 50% formamide at about 37°C to 42°C, whereas hybridization under reduced stringency conditions might employ about 35% to 25% formamide at about 30°C to 35°C. One particular set of conditions for hybridization under high stringency conditions employs 42°C, 50% formamide, 5x SSPE, 0.3% SDS, and 200 µg/ml sheared and denatured salmon sperm DNA. For hybridization under reduced stringency, similar conditions as described above may be used in 35% formamide at a reduced temperature of 35°C. The temperature range corresponding to a particular level of stringency can be further narrowed by calculating the purine to pyrimidine ratio of the nucleic acid of interest and adjusting the temperature accordingly. Variations on the above ranges and conditions are well known in the art. Preferably, hybridization should occur only if there is at least 95%, and more preferably at least 97%, identity between the sequences. The polynucleotides which hybridize to the hereinabove described polynucleotides in a preferred embodiment encode polypeptides which exhibit substantially the same biological function or activity as the mature protein encoded by one of the cDNAs of SEQ ID No. 1-13.

As mentioned, a suitable polynucleotide probe may have at least 14 bases, preferably 30 bases, and more preferably at least 50 bases, and will hybridize to a polynucleotide of the present invention, which has an identity thereto, as hereinabove described, and which may or may not retain activity. For example, such polynucleotides may be employed as a probe for hybridizing to the polynucleotides of SEQ ID No. 1 to 13, respectively, for example, for recovery of such a polynucleotide, or as a diagnostic probe, or as a PCR primer. Thus, the present invention includes polynucleotides having at least a 70% identity, preferably at least a 90% identity, and more preferably at least a 95% identity to a polynucleotide which encodes a polypeptide of SEQ ID No. 1-13, as well as fragments thereof, which fragments preferably have at least 30 bases and more preferably at least 50 bases, and to polypeptides encoded by such polynucleotides.

For example, polynucleotides according to Seq ID No. 14-19 are provided that are used as PCR primers.

Seq ID No. 14 and Seq ID No. 15 comprise oligonucleotides applicable as PCR primers for *ophMA* sequence. In particular, Seq ID No. 14 may be used as forward primer (ophMAfw) and Seq ID No. 15 may be used as reverse primer (ophMArev).

Seq. ID no. 16-19 comprise oligonucleotides applicable as PCR primers for *ophP* sequence. In particular, Seq ID No. 16 may be used as forward primer (ophPfw) of a first *ophP* fragment and Seq ID No. 17 may be used as reverse primer (Sallrev) of the first *ophP* fragment; Seq ID No. 18 may be used as forward primer (Sallfw) of a second *ophP* fragment and Seq ID No. 19 may be used as reverse primer (ophPrev) of the second *ophP* fragment.

The terms "homology" or "identity," as used interchangeably herein, refer to sequence similarity between two polynucleotide sequences or between two polypeptide sequences, with identity being a more strict comparison. The phrases "percent identity or homology" and "identity or homology" refer to the percentage of sequence similarity found in a comparison of two or more polynucleotide sequences or two or more polypeptide sequences. "Sequence similarity" refers to the percent similarity in base pair sequence (as determined by any suitable method) between two or more polynucleotide sequences. Two or more sequences can be anywhere from 0-100% similar, or any integer value there between. Identity or similarity can be determined by comparing a position in each sequence that can be aligned for purposes of comparison. When a position in the compared sequence is occupied by the same nucleotide base or amino acid, then the molecules are identical at that position. A degree of similarity or identity between polynucleotide sequences is a function of the number of identical or matching nucleotides at positions shared by the polynucleotide sequences.

The degree of sequence identity is determined by choosing one sequence as the query sequence and aligning it with the internet-based tool ClustalW with homologous sequences taken from GenBank using the blastp algorithm (NCBI).

As is well known in the art, the genetic code is redundant in that certain amino acids are coded for by more than one nucleotide triplet (codon), and the invention includes those polynucleotide sequences which encode the same amino acids using a different codon from that specifically exemplified in the sequences herein. Such a polynucleotide sequence is referred to herein as an "equivalent" polynucleotide sequence.

The present invention further includes variants of the hereinabove described polynucleotides which encode for fragments, such as part or all of the protein, analogs and derivatives of a polypeptide of SEQ ID No. 1 to 13. The variant forms of the polynucleotide may be a naturally occurring allelic variant of the polynucleotide or a non-naturally occurring variant of the polynucleotide. For example, the variant in the nucleic acid may simply be a difference in codon sequence for the amino acid resulting from the degeneracy of the genetic code, or there may be deletion variants, substitution variants and addition or insertion variants. As known in the art, an allelic variant is an alternative form of a polynucleotide sequence, which may have a substitution, deletion or addition of one or more nucleotides that does not substantially alter the biological function of the encoded polypeptide.

The polynucleotides (and corresponding polypeptides) of the present invention should be in an isolated form, and preferably they are purified to substantial homogeneity or purity. By substantial homogeneity is meant a purity of at least about 85%. In large scale or industrial applications their use in unpurified form is intended, preferably concentrated by removal of liquid.

The term "isolated" is used to mean that the material has been removed from its original environment (e. g., the natural environment if it is naturally occurring). For example, a naturally occurring polynucleotide or polypeptide present in a living organisms not considered to be isolated, but the same polynucleotide or polypeptide, when separated from substantially all of the coexisting materials in the natural system, is considered isolated. For DNA, the term includes, for example, a recombinant DNA which is incorporated into a vector, into an autonomously replicating plasmid or virus, or into the genomic DNA of a prokaryote or eukaryote; or which exists as a separate molecule (e. g., a cDNA or a genomic or cDNA fragment produced by polymerase chain reaction (PCR) or restriction endonuclease digestion) independent of other sequences. It also includes a recombinant DNA, which is part of a hybrid gene encoding additional polypeptide sequence, e.g., a fusion protein. Further included is recombinant DNA which includes a portion of the nucleotides shown in one of SEQ ID No. 1-13.

In a preferred embodiment of the invention, the nucleic acid is a polynucleotide, which has been codon-optimized for recombinant expression in a production host such as yeast.

It is furthermore preferred if a recombinant polynucleotide comprising at least one of the polynucleotide sequence SEQ ID No. 1-13 plus an expression-controlling elements operably linked with said polynucleotide to drive expression thereof is provided.

The object of the present invention is further solved by providing a polypeptide encoded by at least one of the polynucleotides of SEQ ID No. 1 - 13, in particular SEQ ID no. 1, 2, 6-12.

Thus, a Methyltransferase and precursor peptide OphMA, a prolyloligopeptidase OphP, P450-type monooxygenases OphM1, OphM2 and acetyltransferase OphA are provided.

Specifically, methyltransferase and precursor peptide OphMA and prolyloligopeptidase OphP are involved in the biosynthesis of omphalotin A while subsequent steps, assigned to P450 monooxygenases and a putative acetyltransferase perform further decorations of the molecule rendering e.g. omphalotin C or D.

The present invention also refers to vectors, in particular expression vectors, which include such polynucleotides of SEQ ID No. 1-13, and the host cells which are genetically engineered with such vectors. Host cells are genetically engineered (transduced or transformed or transfected) with such vectors, which may be, for example, a cloning vector or an expression vector.

The vector may be, for example, in the form of a plasmid, a conjugative plasmid, a viral particle, a phage, etc. The vector or the gene may be integrated into the chromosome at a specific or a not specified site. Methods for genome integration of recombinant DNA, such as homologous recombination or transposase-mediated integration, are well known in the art. The engineered host cells can be cultured in conventional nutrient media modified as appropriate for activating promoters, selecting transformants or amplifying the genes of the present invention. The culture conditions, such as temperature, pH and the like, are those commonly used with the host cell selected for expression, as well known to the ordinarily skilled artisan.

The polynucleotides of the present invention may be employed for producing polypeptides by recombinant techniques. Thus, for example, the polynucleotides may be included in any one of a variety of expression vectors for expressing polypeptides. Such vectors include chromosomal, nonchromosomal and synthetic DNA sequences, e. g. yeast plasmids; bacterial or yeast artificial chromosome (BAC, YAC); yeast episomal or integrative plasmids (YEps, Ylps) or a vector suitable for fungi. However, any other vector may be used as long as it is replicable and viable in the host, or can be used for genome integration.

The appropriate DNA sequence may be inserted into the vector by any of a variety of procedures. In general, the DNA sequence is inserted into an appropriate restriction endonuclease site (s) by procedures well known in the art, which procedures are deemed to be within the scope of those skilled in this art.

The DNA sequence in the expression vector is operatively linked to an appropriate expression control sequence (s) (promoter) to direct mRNA synthesis.

The expression vector should also contain a ribosome binding site for translation initiation and a transcription terminator. The vector may also include appropriate sequences for amplifying expression. In addition, the expression vectors preferably contain one or more selectable marker genes to provide a phenotypic trait for selection of transformed host cells.

In a most preferred embodiment at least two expression vectors are provided that allow the heterologous expression of at least two of the polynucleotides of Seq ID. No 1-13.

In particular, a first expression vector is provided that enables the expression of a polynucleotide of Seq ID No. 1 or 2 (ophMA) and a second expression vector is provided that enables the expression of a polynucleotide of Seq ID No. 6 or 7 (ophP).

It is however also possible to use one expression vector comprising both ophMA and ophP.

In a preferred embodiment an expression vector for heterologous expression of at least one polynucleotide of SEQ ID. No. 1-13 in yeast, in particular *P. pastoris* or *S*. *cerevisiae,* or in a fungi, in particular Aspergillus sp., such as *A. niger* is provided.

The preferred expression vectors are suitable for heterologous expression of the present polynucleotides / polypeptides in *P. pastoris.* Such vector may comprise a constitutive, inducible or repressible promoter that can switch on or switch off the polypeptide expression and thus the biosynthesis of omphatolin. Such a suitable promoter may be a methanol inducible promoter such as AOX promoter and is integrated into the genome for chromosomal expression.

An example for *P. pastoris* expression vector may be expression vector pPIC3.5 K (Thermo Fisher Scientific). The pPIC3.5K vector carries the kanamycin resistance gene. *Pichia* transformants are selected on histidine-deficient medium. Another suitable expression vector for heterologous expression in *P. pastoris* may be the shuttle vector pPICHOLI-2 (MoBiTech).

In case of *S*. *cerevisiae* a Galactose induced system may be used as expression vector or expression system.

As mentioned previously, a host cell that has been genetically engineered by the insertion of a polynucleotide of SEq ID No. 1-13 or the vector containing said polynucleotides is also provided.

The vector containing the appropriate DNA sequence as hereinabove described, as well as an appropriate promoter or control sequence, may be employed to transform the appropriate host to permit the host to express the protein. As representative examples of appropriate hosts, there may be mentioned yeast such as *Pichia, Saccharomyces or Aspergillus, P. pastoris* and *S. cerevisiae* and *A. niger* being the most preferred expression hosts.

In a preferred embodiment, at least one expression vector comprising *ophMA* and at least one second expression vector comprising *ophP* are transformed into the host cell. The vectors may be integrated into the chromosome of the host cell or may be present in the host cell as self-replicating vectors. A chromosomal integration is however mostly preferred.

The object of the present invention is also solved by providing a process for producing at least one omphalotin compound comprising the steps of culturing the host cell containing at least one of the polynucleotides of SEQ ID. No 1-13 under conditions suitable for the biosynthesis of the at least one omphalotin compound.

It is preferred that the biosynthesis of the at least one omphalotin compound is induced by adding at least one suitable inducer to the culture medium.

In a preferred embodiment of the present process the host cell comprises at least one expression vector (or chromosomal expression cassette) with the *ophMA* encoding sequence and at least one expression vector (or chromosomal expression cassette) with the *ophP* encoding sequence. The expression of both enzymes OphMA and OphO may be induced by the same or different inducers. The expression of both enzymes enables the biosynthesis of at least one omphalotin compound, in particular omphalotin A.

The present process may further include the step of removing and isolating the at least one omphalotin compound from the culture medium.

The at least one omphatolin compound can be recovered and purified from recombinant cell cultures by methods including salt or solvent precipitation, acid extraction, ultra-filtration, anion or cation exchange chromatography, phosphocellulose chromatography, hydrophobic interaction chromatography, size exclusion chromatography, affinity chromatography, hydroxylapatite chromatography, lectin chromatography and ultrafiltration.

High performance liquid chromatography (HPLC) can be employed for final purification steps. In special cases purification may not be necssary.

The present invention is further explained in detail by means of the following examples with reference to the figures. It shows:
- Figure 1: a general overview of the gene cluster involved in the omphalotin biosynthesis,
- Figure 2: ion chromatograms of omphalotin A produced in *O. olearius;*
- Figure 3: ion chromatograms of omphalotin A intermediate stages produced in *P*. *pastoris;*
- Figure 4: an MS chromatogram of omphalotin A produced by *P. pastoris,* and
- Figure 5: an MS chromatogram of linear omphalotin A produced by *P. pastoris,* and

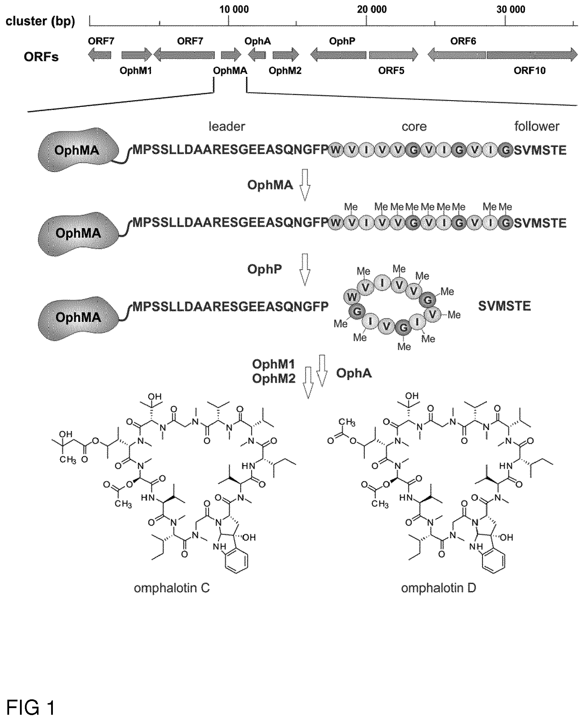

The scheme of Figure 1 illustrates the gene cluster involved in omphatolin biosynthesis.

The two enzymes, OphMA and OphP, are required to reconstitute the biosynthesis of omphalotin A. Specifically, am automethylation of methyltransferase OphMA is followed by processing of the prolyl oligopeptidase OphP. Subsequent steps introduce hydroxylations (OphM1 and OphM2 as P450 monooxygenases) and acylations by an acetyltransferase (OphA), rendering omphalotin C and D.

Higher multiply methylated linear omphalotin precursor peptides may also be detected and may be interpreted as the occurrence of shunt products, which may have hydrolyzed from an overwrought or a partially incorrectly folded cyclizing protease OphP. Nevertheless, multiple methylations appear to be a precondition for the action of OphP, which likely forms a peptidylester upon removal of the follower peptide which is then macrocyclized with the N-terminus of the modified core peptide.

The present invention represents an initial steps of the omphaplotin biosynthesis, which is characterized by some unusual features: firstly, the striking structural similarity between omphalotin and cyclosporine initially let assume a non-ribosomal peptide assembly. Secondly, multiple methylations of the peptide backbone thus far have only been described for NRPS. Thirdly, the key-enzyme OphMA appears to be an automodifying methyltransferase, which requires the subsequent processing by a protease (OphP), a biosynthesis mechanism, which is unprecedented in RiPPs.

### Example 1: Materials and Equipment

All chemicals and antibiotics were purchased from Carl Roth GmbH & Co or Sigma Aldrich and used without further purification unless otherwise specified. Messenger RNA was extracted from *Omphalotus olearius* strain DSM-3398 (DMSZ -Deutsche Sammlung von Mikroorganismen und Zellkulturen GmbH, Leibniz-Institut, Braunschweig, Germany) using TRIzol® reagent and translated into cDNA with the *First Strand cDNA* Kit from Thermo Fisher Scientific. *Escherichia coli cells* DH5α, the overexpression host *Pichia pastoris* GS115, DNA modifying enzymes (for PCR amplification, restriction digest and ligation) were obtained from Thermo Fisher Scientific as well as the pPIC3.5 vector. The vector pPICHOLI-2 was purchased from MoBiTec. All primers were ordered from Thermo Fisher Scientific and the DNA sequencing was carried out by Eurofins Genomics (Munich, Germany).

### Example 2: Preparation and Purification of DNA and RNA

### mRNA Extraction and cDNA Synthesis

Genes encoding OphMA and OphP were amplified from complementary DNA (cDNA). Therefore messenger RNA (mRNA) from omphalotin producing *Omphalotus olearius* submerse cultures was extracted. A culture of *O*. *olearius* was grown for 7 days (28 °C; 80 rpm) on MEA-medium (30 g malt extract and 3 g soya peptone per liter). Grown colonies were crushed in a mortar and transferred into YMG-medium (10 g malt extract, 4 g yeast extract and 4 g glucose per liter). After an additional incubation of 10 days (28 °C; 80 rpm) *O*. *olearius* started to produce Omphalotin. As control for the production of Omphalotin the dodecapeptide was isolated from the supernatant.

Detection of omphalotin was performed by means of HPLC-Orbitrap mass spectrometry (MS) (see S3.2). For the extraction of mRNA with the TRIzol® reagent (Thermo Fisher Scientific) a highly omphalotin producing culture (judged by MS detection) was used and executed according to the manufacturer's instructions. The mRNA was translated into cDNA with the First Strand cDNA Kit (Thermo Fisher Scientific) according the manufacturer's instructions and used as a template for the PCR amplification of *ophMA* and *ophP.*

### Cloning of ophMA and ophP for Pichia pastoris

Genes encoding OphMA (methyltransferase + precursor peptide) and OphP (prolyloligopeptidase) of the basidiomycete *O*. *olearius* were first amplified by means of PCR from cDNA (Q₅ polymerase, NEB) and cloned into the vectors pET28a(+) (*ophMA)* and pET-trx-1 b *(ophP).* These plasmids were used as template for the amplification and cloning into the vector pPICHOLI-2 (MoBiTech) (*ophMA*) and vector pPIC3.5K (Thermo Fischer Scientific) (*ophP*).

Used primers are listed in table S1. For *ophP* two sets of primers were used in two different PCR reactions to remove a Sall restriction site (silent mutation). Each PCR reaction (50 µL total volume) consisted of 35 µL sterile *dd*H₂O, 10 µL 5x Q₅ reaction buffer, 1 µL of 10 µM dNTP solution, 1 µL of each primer (forward and reverse) at 10 µM, 1 µL template and 1 µL of Q₅ polymerase. The PCR reaction was carried out using a T Gradient thermocycler (Biometra). Lid temperature was set to 100 °C and the initial denaturation was at 98 °C for 1 min. The amplification included a 10 s denaturation at 98 °C, primer annealing at 68 °C for 10 s and an elongation step at 72 °C for 2 min. The last three steps were repeated 35 times and a final elongation at 72 °C for 5 min followed.

The resulting PCR products were purified with an agarose gel electrophoresis and PCR products were excised and purified using a Gel extraction Kit (Thermo Fisher Scientific) according to manufacturer's instructions. Then the PCR product containing *ophMA* was digested with SgrDI and Notl, pPICHOLI-2 was digested with Sall and Notl, pPIC3.5K was digested with Sall. Subsequently both plasmids as well as the digested PCR product were purified as described for the PCR products. Both fragments of *ophP* were cloned into the pPIC3.5K vector using Gibson Assembly® Master Mix (NEB) according the manufacturer's instructions. The gene containing *ophMA* was cloned with T4 ligase into the pPICHIOLI-2 vector. Thus generated plasmids were used to transform *E. coli* DH5α cells (Thermo Fischer Scientific) according the manufacturer's instructions. Cells were spread on LB-Agar plates (per liter: 5 g yeast extract, 10 g tryptone, 5 NaCl and 15 g Agar) supplemented with respective antibiotics and incubated over night at 37 °C. Single colonies were picked and used to inoculate 20 ml LB medium (per liter: 5 g yeast extract, 10 g tryptone and 5 NaCl) supplemented with respective antibiotics. Cells were cultured overnight at 37 °C and 180 rpm supplemented with respective antibiotics.

Plasmids were isolated using the Plasmid Extraction Kit (Thermo Fischer Scientific) according the manufacturer's instructions. The correct insertion of inserts and DNA sequences was verified by sequencing (Eurofins Genomics). For generation of the heterologous Pichia producer, *P. pastoris* GS115 cells were first transformed with pPIC3.5K (Thermo Fisher Scientific) bearing *ophP* according to manufacturer's instructions and in a second step with pPICHOLI-2 (MoBiTech) bearing *ophMA* according to manufacturer's instructions.

**Table 1: Used Oligonucleotides and Gene Sequences**

| name | Sequence 5' → 3' | Usage |
|---|---|---|
| *ophMA* fw | CGTCGACGAGAACTTGTATTTCCAGGAGACTTCCAC TCAGACC | *ophMA* |
| *ophMA* rev | GCGGCCGCATTATTCCGTGCTCATGACTGATCC | *ophMA* |
| *ophP* fw | CTAATTATTCGAAGGATCCTACATGAGCGATAAAATT ATTCA | first fragment *ophP* |
| Sall rev | AGAACGAAAATATCGTTATCCTGCCGTCGTCCTGATA CCA | first fragment *ophP* |
| Sall fw | TGGTATCAGGACGACGGCAGGATAACGATATTTTCG TTCT | second fragment *ophP* |
| *ophP* rev | GGCCGCCCTAGGGAATTCTACTCAAACTGTTACGCA GGACC | second fragment *ophP* |

### Example 3: Purification and Analytical Characterization of Omphalotin

### Omphalotin Extraction from Submerse Cultures of O. olearius and P. pastoris

### Omphalotus olearius

For isolation and detection of omphalotin from *O*. *olearius* 2 mL supernatant of an omphalotin producing submerse culture (see S2.1) were vigorously mixed for 1 min with the same volume ethyl acetate. The organic phase was separated and vaporized. The resulting pellet was re-suspended in 37 % methanol to a final concentration of 0.1 mg /ml and 5 µl were used for HPLC-Orbitrap-MS analysis (see S3.2).

### Pichia pastoris

For isolation and detection of omphalotin from *P. pastoris* GS115 a strain carrying *ophP* integrated into the genome which also carries the pPICHOLI-2 plasmid containing *ophMA* was used (see S2.2). *P. pastoris* GS115 cells were pre-cultured overnight at 30 °C in 40 ml YPD-medium (per liter: 20 g peptone, 10 g yeast extract and 20 g glucose) at 160 rpm. The main culture was inoculated with an OD₆₀₀ 0.25 in 500 ml BMMY-medium (per liter: 10 g yeast extract, 10 g trypton, 13.4 g YNB and 100 ml 1 M potassium phosphate buffer adjusted to pH 6.0) and further cultivated at 30 °C and 160 rpm to OD₆₀₀ 1.0 after which gene expression was induced by addition of 0.5% (v/v) methanol. Cells were further incubated at 30 °C and 160 rpm for 72 h and after 24 h and 48 h 0.5 % (v/v) methanol was added.

Cells and supernatant (500 ml culture) was separated by centrifugation (30 min, 4 °C, 10,000 g). Supernatant was strongly mixed for 1 h with the same volume hexanes. The pellet was re-suspended in 40 ml buffer (300 mM NaCl and 50 mM Tris adjusted to pH 8.0) and disrupted with a French press (TS-series 0,75 kW, Constant systems) at 40 kPsi. Cells debris was centrifuged (60 min, 4 °C, 75,000g) and the supernatant was strongly mixed for 1 h with the same volume of hexanes.

The organic phase was separated and vaporized. The resulting pellets were re-suspended in aqueous methanol (37 %) to a final concentration of 0.1 mg /ml and 5 µl were used for HPLC-Orbitrap-MS (see S3.2).

### HPLC-ESI-MS Detection of Omphalotin from Submerse Cultures of O. olearius and P. pastoris

Detection of omphalotin was conducted using an ESI-Orbitrap-MS (Exactive, Thermo Fisher Scientific) coupled to a HPLC system 1200 (Agilent Technologies). Chromatographic separation were performed using a Poroshell 120 EC.C18 2.7 µm column (50 x 2.1 mm, Agilent Technologies) with a linear mobile phase gradient consisting of solvent A (0.1% (v/v) formic acid in water and solvent B (0.1 % (v/v) formic acid in acetonitrile). The gradient was 37-100% B over 11 min. Data was analyzed using the Thermo Xcalibur 2.2 software.

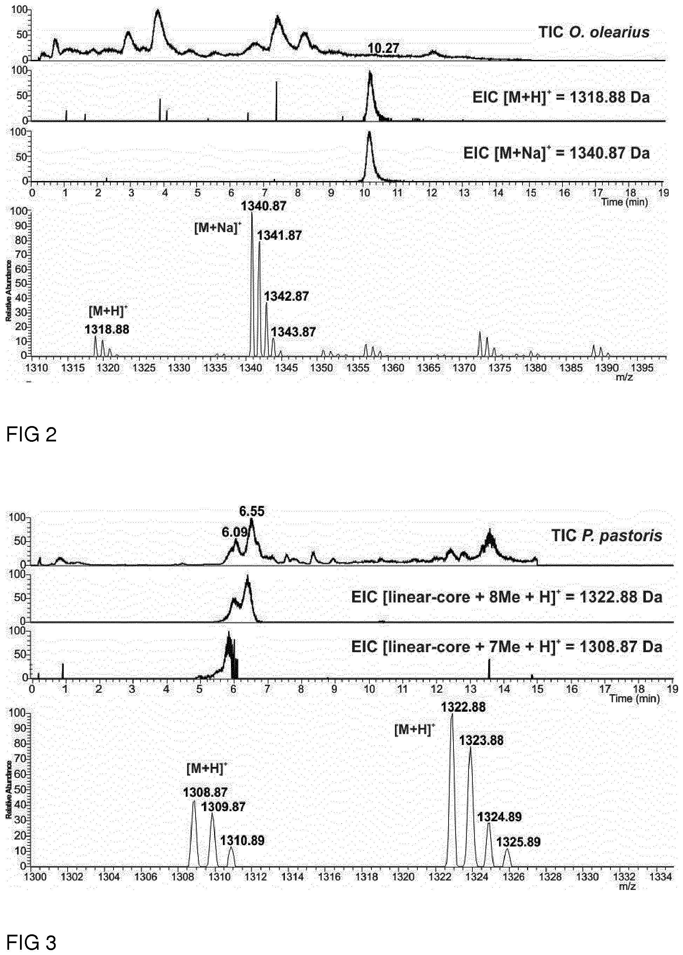

The production of omphalotin A in *O*. *olearius* was analyzed by ion chromatography (see Figure 2). Total-ion chromatogram (TIC) and extracted-ion chromatogram (EIC) of the cyclized peptide [M+H]⁺= 1318.88 Da and [M+Na]⁺ = 1340.87 Da measured by ESI-Orbitrap-MS.

The production of omphalotin A intermediate stages in *P. pastoris* was also analyzed by ion chromatography (see Figure 3). Total-ion chromatogram (TIC) and extracted-ion chromatogram (EIC) of the linear core peptide 8x methylated [linear-peptide + 8Me + H]⁺= 1322.88 Da and the linear core peptide 7x methylated [linear peptide + 7 Me + H]⁺ = 1308.87 Da measured by ESI-Orbitrap-MS.

### HPLC-ESI-MS / MS Detection of Omphalotin from Submerse Cultures of O. olearius and P. pastoris

All HPLC-ESI-MS/MS analyses were conducted using an ESI-Triple-Quadropol-MS (6460 Series, Agilent Technologies) coupled to a HPLC system 1200 (Agilent Technologies). Chromatographic separations were performed using Grom-Sil 300 Octyl-6MB 2.3 µm columns (50 x 2.3 mm, Grace) with a linear mobile phase gradient consisting of solvent A (0.1% (v/v) formic acid in water and solvent B (0.1 % (v/v) formic acid in acetonitrile). The gradient was 37-100% B over 6 min. Data was analyzed using the MassHunter Qualitative Analysis B.07.00 software.

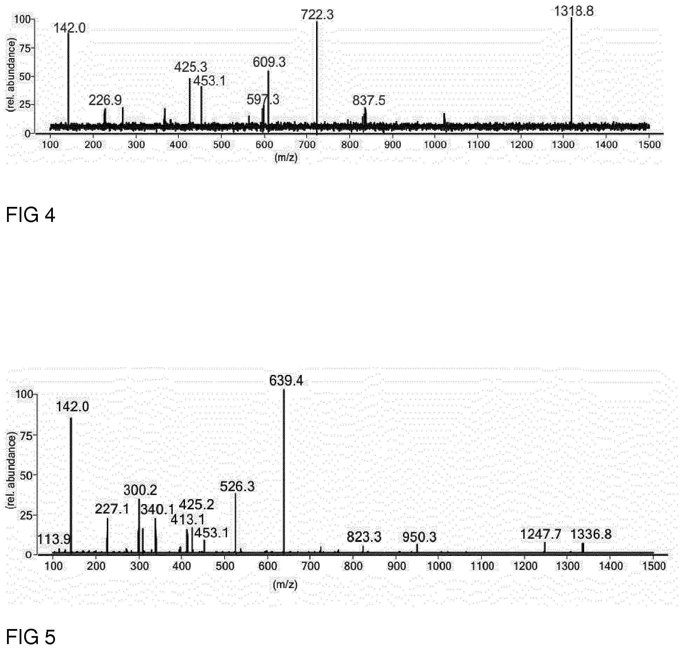

Figure 4 and Table 2 shows the characteristic and diagnostic fragments of omphalotin A produced by *P. pastoris* by tandem MS measurements.

**Table 2: Observed and identified fragments of omphalotin A after tandem MS measurements**

| observed mass | expected mass |
|---|---|
| 142.0 | no match found |
| 226.9 | 227.3 |
| 425.3 | 425.3 |
| 453.1 | 453.3 |
| 597.3 | 597.4 |
| 609.3 | 609.4 |
| 722.3 | 722.5 |
| 837.5 | 837.6 |
| 1318.8 | 1318.9 |

Figure 5 and Table 3 show the structural characterization of linear omphalotin A produced by *P. pastoris* by tandem MS measurements.

**Table S3. Observed and identified fragments of linearomphalotin A after tandem MS measurements**

| **observed mass** | **expected mass** |
|---|---|
| 113.9 | 114.1 |
| 142.0 | no match found |
| 227.1 | 227.2 |
| 300.2 | 300.2 |
| 340.1 | 340.3 |
| 413.1 | 413.3 |
| 425.2 | 425.3 |
| 453.1 | 453.3 |
| 526.3 | 526.4 |
| 639.4 | 639.4 |
| 823.3 | 823.5 |
| 950.3 | 950.6 |
| 1247.7 | 124.9 |
| 1336.8 | 1336.9 |

**Table 3**: Observed and identified fragments of linearomphalotin A after tandem MS measurements

## Claims

1. A polynucleotide encoding for a polypeptide, in particular with N-methyltransferase activity and omphatolin precursor peptide activity, consisting essentially of Seq ID No. 1 or 2 or a polynucleotide with at least 70%, preferably at least 90%, more preferably at least 95 % identity thereto.

2. A polynucleotide encoding for a polypeptide, in particular with prolyloligopeptidase activity, consisting essentially of Seq ID No. 6 or 7 or a polynucleotide with at least 70%, preferably at least 90%, more preferably at least 95 % identity thereto.

3. A polynucleotide encoding for a polypeptide, in particular with monooxygenase activity, consisting essentially of Seq ID No. 8 or a polynucleotide with at least 70%, preferably at least 90%, more preferably at least 95 % identity thereto.

4. A polynucleotide encoding for a polypeptide, in particular with monooxygenase activity, consisting essentially of Seq ID No. 9 or 10 or a polynucleotide with at least 70%, preferably at least 90%, more preferably at least 95 % identity thereto.

5. A polynucleotide encoding for a polypeptide, in particular with acetyltransferase activity, consisting essentially of Seq ID No. 11 or 12 or a polynucleotide with at least 70%, preferably at least 90%, more preferably at least 95 % identity thereto.

6. A polynucleotide encoding for polypeptides with activity for omphalotin synthesis comprising at least one of the Seq ID No. according to one of claims 1-5, and consisting essentially of Seq ID No. 13 or a polynucleotide with at least 70%, preferably at least 90%, more preferably at least 95 % identity thereto.

7. A recombinant polynucleotide comprising the polynucleotide sequence according to one of the claims 1-6 plus expression-controlling elements operably linked with said polynucleotide to drive expression thereof.

8. A polypeptide encoded by at least one of the polynucleotides according to one of the claims 1 to 5.

9. Expression vector comprising at least one polynucleotide according to one of the claims 1-7.

10. Expression vector according to claim 9 for heterologous expression of at least one polynucleotide according to claims 1-7 in yeast, in particular in Pichia or Saccharomyces, or in fungi, in particular in Aspergillus.

11. A host cell that has been genetically engineered by the insertion of a polynucleotide according to one of the claims 1 to 7 or the vector of claim 9.

12. Host cell according to claim 11, wherein the host cell is selected from yeast, in particular from Pichia or Saccharomyces, or a fungi, in particular Aspergillus.

13. Process for producing at least one omphalotin compound comprising the step of culturing the host cell of claims 11 or 12 under conditions suitable for the biosynthesis of the at least one omphalotin compound.

14. Process according to claim 13, wherein the biosynthesis of the at least one omphalotin compound is induced by adding at least one suitable inducer to the culture medium.

15. Process according to one of the claims 13 to 14, further including the step of removing and isolating the at least one omphalotin compound from the culture medium.


SEQUENCE LISTING

<110>  Techische Universität Berlin

<120>  Polynucelotides encoding for polypeptides involve in omphatolin
       biosynthesis

<130>  TU137EP

<160>  22

<170>  PatentIn version 3.5

<210>  1
<211>  1468
<212>  DNA
<213>  Omphalotus olearius


<220>
<221>  ophMA
<222>  (1)..(1468)
<223>  chromsomal DNA

<400>  1
atgagaggat cgcatcacca tcaccatcac ggatccctga acgacatctt cgaagctcag      60

aaaatcgaat ggaaaaaggt cgacgagaac ttgtatttcc aggagacttc cactcagacc     120

aaagctggct cactcaccat cgtcggtacc ggtatcgaga gtatcggaca aatgacgctt     180

caggcgttgt cctacatcga agccgccgcg aaggttttct actgcgtcat cgaccccgcc     240

actgaggcat tcatcctcac caagaacaag aactgcgttg acttgtatca gtattacgac     300

aatggcaagt ccagattgaa cacttacacc caaatgtcag aggtaagctt cgtacaccct     360

caacagtcgc ccacaccccg acgctgacac acgcatagct catggtcagg gaagtccgca     420

agggcctcga tgtcgtgggc gtcttctacg gccacccagg agtgttcgtg aacccgtctc     480

accgagctct ggctatcgcc aagagtgaag gctaccgagc gaggatgctt ccaggcgtgt     540

ctgcggaaga ttgtctcttc gcggacttgt gtatcgatcc ttcgaacccg ggttgcctga     600

cctacgaggc atcggatttc ctgatcaggg atcgcccggt cagcatccac agtcacttgg     660

tcctgttcca agtcggatgc gtcggtattg ctgactttaa cttcactgga ttcgacgtaa     720

gttctttcac tccaatagct caagatcttc cgcttacctc ctcacgaatg gccagaacaa     780

caaattcggc gttctcgtcg accgtctcga gcaagaatac ggcgccgagc accctgtcgt     840

ccattacatc gcagctatga tgccacacca agacccagtc accgataagt acaccgtcgc     900

gcagctccgt gagcccgaga tcgcgaagcg tgttggcggt gtctcgactt tctacattcc     960

tcccaaggcc aggaaagcat cgaacttgga catcataagg cgcctagagc tcttgcctgc    1020

tgggcaagtt cccgacaaga aagcgcgtat ttacccggcc aaccagtggg agcccgatgt    1080

tcccgaagtc gaaccctaca gaccatctga ccaggctgcc atcgctcagt tggctgacca    1140

cgctcctcct gagcaatatc aacctctcgc tacttcgaaa gccatgtctg atgttatgac 1200

gaagttggct ttggatccaa aggcactcgc cgactacaaa gctgatcacc gcgcctttgc 1260

tcaatctgtc cccgacttga cgcctcagga gcgtgcggct ttggagctcg gtgattcgtg 1320

ggctattcgt tgcgcgatga agaatatgcc ctcgtcgctc ttggacgctg ctcgtgaatc 1380

cggcgaagag gcatcccaaa acggtttccc atgggtcatc gtcgttggtg tcatcggtgt 1440

catcggatca gtcatgagca cggaataa 1468

<210> 2
<211> 1257
<212> DNA
<213> Omphalotus olearius

<220>
<221> ophMA_cDNA
<222> (1)..(1257)

<400> 2
atgggcgaga cttccactca gaccaaagct ggctcactca ccatcgtcgg taccggtatc 60

gagagtatcg gacaaatgac gcttcaggcg ttgtcctaca tcgaagccgc cgcgaaggtt 120

ttctactgcg tcatcgaccc cgccactgag gcattcatcc tcaccaagaa caagaactgc 180

gttgacttgt atcagtatta cgacaatggc aagtccagat tgaacactta cacccaaatg 240

tcagagctca tggtcaggga agtccgcaag ggcctcgatg tcgtgggcgt cttctacggc 300

cacccaggag tgttcgtgaa cccgtctcac cgagctctgg ctatcgccaa gagtgaaggc 360

taccgagcga ggatgcttcc aggcgtgtct gcggaagatt gtctcttcgc ggacttgtgt 420

atcgatcctt cgaacccggg ttgcctgacc tacgaggcat cggatttcct gatcagggat 480

cgcccggtca gcatccacag tcacttggtc ctgttccaag tcggatgcgt cggtattgct 540

gactttaact tcactggatt cgacaacaac aaattcggcg ttctcgtcga ccgtctcgag 600

caagaatacg gcgccgagca ccctgtcgtc cattacatcg cagctatgat gccacaccaa 660

gacccagtca ccgataagta caccgtcgcg cagctccgtg agcccgagat cgcgaagcgt 720

gttggcggtg tctcgacttt ctacattcct cccaaggcca ggaaagcatc gaacttggac 780

atcataaggc gcctagagct cttgcctgct gggcaagttc ccgacaagaa agcgcgtatt 840

tacccggcca accagtggga gcccgatgtt cccgaagtcg aaccctacag accatctgac 900

caggctgcca tcgctcagtt ggctgaccac gctcctcctg agcaatatca acctctcgct 960

acttcgaaag ccatgtctga tgttatgacg aagttggctt tggatccaaa ggcactcgcc 1020

gactacaaag ctgatcaccg cgcctttgct caatctgtcc ccgacttgac gcctcaggag 1080

cgtgcggctt tggagctcgg tgattcgtgg gctattcgtt gcgcgatgaa gaatatgccc 1140

tcgtcgctct tggacgctgc tcgtgaatcc ggcgaagagg catcccaaaa cggtttccca 1200

tgggtcatcg tcgttggtgt catcggtgtc atcggatcag tcatgagcac ggaataa 1257

<210> 3
<211> 66
<212> DNA
<213> Omphalotus olearius

<220>
<221> leader
<222> (1)..(66)

<400> 3
atgccctcgt cgctcttgga cgctgctcgt gaatccggcg aagaggcatc ccaaaacggt 60

ttccca 66

<210> 4
<211> 36
<212> DNA
<213> Omphalotus olearius

<220>
<221> core
<222> (1)..(36)

<400> 4
tgggtcatcg tcgttggtgt catcggtgtc atcgga 36

<210> 5
<211> 18
<212> DNA
<213> Omphalotus olearius

<220>
<221> follower
<222> (1)..(18)

<400> 5
tcagtcatga gcacggaa 18

<210> 6
<211> 3905
<212> DNA
<213> Omphalotus olearius

<220>
<221> ophP
<222> (1)..(3905)
<223> chromosomal DNA

<400> 6
atggaagagg atgtctttct taacgacatc aatttgttgg gggatttcca tcacctgggg 60

gcagcggata agtaagtaac agtccgtgca gcatcgtctg tacgccactc ccgctgtcag 120

aatgtcaaac ccttttctcg tgattacttg tttgagtaca tactttctcc tcctcgtctt 180

agagttccag aataacggca atcattagac gatgcatgcg aaagaccctg atctatctta 240

gcaggtcagc tgcacaatcg tgaatctaca tatactcatt gattcgtacc gatccgcgtt 300

ggcattggaa atgttggggc caagtatggt actcagtctt tcactctgct tccgttaata 360

cgaatctcat cagacgtgat ctgggtttcg tgtgctatga cggaaccttt agtcttatca 420

ccgaatggag taacgagatc gcacgatgtt caggagaaca cactgctcct cgagattgaa 480

gtggatacaa tcgacgtcgc tgatcttctt cttgtgctca gttgtaggac gatgtcgttt 540

ccaggatggg gaccatatcc acctgttgaa agggacgaaa cttcagcaat aacttacagt 600

agcaagttgc atggctctgt tacggttcgt gatccctaca gtcaacttga agtacctttc 660

gaggatagtg aagaaacgaa ggttcgtgaa cgattccgca attcttacac cttattctga 720

atggcacgaa caggcgtttg ttcattcaca acgcaagttt gcacgaacct atctagatga 780

aaatcccgat cggtaggtat cctcgagttt tctggcccac tttgacctta ctgcagtcct 840

tttacagaga agcatggctc gagacgctga agaagagctg gaattacagg agatttagcg 900

cattgaaacc tgaaagcgac ggacattact acttcgagta taacgatggc cttcaatctc 960

agctttccct gtatcgtgtg aggatggggg aggaggatac tgtcctcact gagtctggtc 1020

ctggtggaga actctttttc aatcctaact tgctctccct tgatggcaac gccgcactta 1080

ctggtttcgt catgagtcca tgtgggaatt attgggcata tggtgtatct gaacatgttc 1140

gtatctatct cataatgcat tcggacgtta ctcataggac gtcacaggga tccgactgga 1200

tgtctattta cgttcgaaag acgtcgtctc cgcatcttcc gtcacaagaa cgaggaaagg 1260

atcctggacg gatgaacgac aagattcgac acgtccgttt cttcattgta tcttggacta 1320

gcgacagcaa aggtcaattc tcaacgattg aagatgagga attgaactag atggcgtttg 1380

cgttaggttt tttctattct aggtatcctc ctgaggatga cgaaggaaaa ggtaatgcac 1440

cagcaatgaa ttgcatggtg agattctttt gatcgtagtt tatcttgttc ttgatgcgtc 1500

tatcaggtct attatcatcg gattggcgag gatcaggaga gtgacgtcct tgttcagtga 1560

gtactctatg tcgaatgatg ccgatctcaa atgttgaaga gtcttcagtg aggatcctga 1620

gcatccgttc tggatctcgt cggtgcagct cactccgagg taggctgtgt ttcttcataa 1680

acgcgctatg ctaattcgtt gtttatgtag cggccgatac atcctctttg ccgcgagcgt 1740

gcgtctggtt caattcacac cgctatcagt tctctaaaga tgcgtgcagc gcgacgcaag 1800

tcacactcaa ttagtcaaga tcgcagatct tcacgaaaat gacattggga cgaatatgaa 1860

atggaaaaac cttcatgatc catgggaggc taggtttaca atgttcgtaa gatctgaggt 1920

tctaggtatt caattactta ctaccatact ggcagagtcg gagacgaggg ttcaaagatc 1980

tatttcatga ccaatctcaa ggccaagaac tacaaagtgg cgacgtttga tgccaaccac 2040

ccagacgagg gtctgacgac gctcatagca gaagacccta acgcgtttct cgtttcagca 2100

agcattcacg cccaagacaa gctcctactt gtttatcttc gtaatgctag ccacgagatt 2160

catatccgtg atctaaccac tgggaaaccc ctcgggcgta tctttgagga tttacttgga 2220

cagtttatgg tatcaggtcg acggcaggat aacgatattt tcgttctctt tagcagtttc 2280

ctctctcctg gaactgtgta tcggtacgta gtagtcgtgg aacccttctg ttacccggac 2340

tgatgcagag cccgaccagg tacacgttcg gtgaagagaa aggacattct tcactatttc 2400

gtgctataag tattccgggc ctcaatctag atgactttat gactgagtcg gtgaggtcaa 2460

ttataccatc ccatcgcgct tgcgactgaa ataccatgat caggttttct atccatccaa 2520

ggacgggact tcgtaattac tacaacttca caaaaaatca cagaggacta acaaatcacg 2580

tagtgttcac atgtttatca cccgcccgaa ggatgtactg ctcgatggaa cttcccctgt 2640

cctacaatac ggctatggcg gtttctccct agcaatgctt cctacattct ccctctctac 2700

gctgctattc tgcaaaattt acagggcgat ctatgcgata cccaatattc ggtgatccat 2760

caagcgttca gattgctgtc ctaatgctga cctactttag tggtggttcg gaatacggcg 2820

agtcatggca ccgggaggtg agtctatgtc ggtctgatga tcgatgatac atgttttgct 2880

gagcgttatc cagggtatgt tagacaagaa gcagaacgtg tttgacgact tcaacgctgc 2940

aaccgaatgg cttattgcga ataagtacgc aagcaaggat cgcattgcca tccgaggagg 3000

atcaaatgga ggtgcgaaac ctattaccta gctgaagtta attccgatct ctgaatagta 3060

tccgatgttc gcaggtgttt tgaccactgc atgtgcaaat caagcaccgg gactctaccg 3120

ttgtgtgatc acaattgagg gtataattga tatgcttaga gtcagttgac tacattctga 3180

acttgctcat tgtcttgctc actaattcct gtgtcaccag tttcctaagt ttacatttgg 3240

cgctagctgg cgttctgaat atggcgatgt acgaatacct acacttacta agtatgcgat 3300

gattctgacg gtcgcctaac ggttgtagcc tgaggatcct gaagatttcg acttcatatt 3360

caagttagtc aatctactat attgactttt cgtcactcaa gataggactt tctagatact 3420

ccccctatca caacatccct cccccgggag acactattat gccagccatg ctattcttta 3480

ctgcggcgta tgatgatcgt gtctcgcctt tgcacagtag gttcaacaga ggttcgatgg 3540

aatcgtatac tttacttacc cttacgcagc gttcaagcac gttgcggcat tgcaacacaa 3600

ctttcccaaa ggcccgaatc catgcctaat gcgcatcgac ttgaattcgg gacactttgc 3660

tggcaaggta tgcattattg gatggcggta tttacactat ttgcttatta tcgtccttgt 3720

agagtacgca ggagatgttg gaagaaacgg cagatgaata caggtacgtc cagcgacgaa 3780

atgtaagggc tctctgtatt taactttctt agcttcatcg ggaagtctat gggcctcact 3840

atgcagactc aaggttcagt agattcaagc cgctggtcct gcgtaacagt ttgaaaagcg 3900

tttga                                                              3905

<210>  7
<211>  2463
<212>  DNA
<213>  Omphalotus oelarius

<220>
<221>  ophP_cDNA
<222>  (1)..(2463)

<400>  7
atgggccgtg aatctatact cattgattcg taccgatccg cgttggcatt ggaaatgttg      60

gggccaaacg tgatctgggt ttcgtgtgct atgacggaac ctttagtctt atcaccgaat     120

ggagtaacga gatcgcacga tgttcaggag aacacactgc tcctcgagat tgaagtggat     180

acaatcgacg tcgctgatct tcttcttgtg ctcagttgta ggacgatgtc gtttccagga     240

tggggaccat atccacctgt tgaaagggac gaaacttcag caataactta cagtagcaag     300

ttgcatggct ctgttacggt tcgtgatccc tacagtcaac ttgaagtacc tttcgaggat     360

agtgaagaaa cgaaggcgtt tgttcattca caacgcaagt ttgcacgaac ctatctagat     420

gaaaatcccg atcgagaagc atggctcgag acgctgaaga agagctggaa ttacaggaga     480

tttagcgcat tgaaacctga aagcgacgga cattactact tcgagtataa cgatggcctt     540

caatctcagc tttccctgta tcgtgtgagg atgggggagg aggatactgt cctcactgag     600

tctggtcctg gtggagaact ctttttcaat cctaacttgc tctcccttga tggcaacgcc     660

gcacttactg gtttcgtcat gagtccatgt gggaattatt gggcatatgg tgtatctgaa     720

catggatccg actggatgtc tatttacgtt cgaaagacgt cgtctccgca tcttccgtca     780

caagaacgag gaaaggatcc tggacggatg aacgacaaga ttcgacacgt ccgtttcttc     840

attgtatctt ggactagcga cagcaaaggt tttttctatt ctaggtatcc tcctgaggat     900

gacgaaggaa aaggtaatgc accagcaatg aattgcatgg tctattatca tcggattggc     960

gaggatcagg agagtgacgt ccttgttcat gaggatcctg agcatccgtt ctggatctcg    1020

tcggtgcagc tcactccgag cggccgatac atcctctttg ccgcgagccg cgacgcaagt    1080

cacactcaat tagtcaagat cgcagatctt cacgaaaatg acattgggac gaatatgaaa    1140

tggaaaaacc ttcatgatcc atgggaggct aggtttacaa tagtcggaga cgagggttca    1200

aagatctatt tcatgaccaa tctcaaggcc aagaactaca aagtggcgac gtttgatgcc    1260

aaccacccag acgagggtct gacgacgctc atagcagaag accctaacgc gtttctcgtt    1320

tcagcaagca ttcacgccca agacaagctc ctacttgttt atcttcgtaa tgctagccac    1380

gagattcata tccgtgatct aaccactggg aaacccctcg ggcgtatctt tgaggattta    1440

cttggacagt ttatggtatc aggacgacgg caggataacg atattttcgt tctctttagc 1500

agtttcctct ctcctggaac tgtgtatcgg tacacgttcg gtgaagagaa aggacattct 1560

tcactatttc gtgctataag tattccgggc ctcaatctag atgactttat gactgagtcg 1620

gttttctatc catccaagga cgggacttct gttcacatgt ttatcacccg cccgaaggat 1680

gtactgctcg atggaacttc ccctgtccta caatacggct atggcggttt ctccctagca 1740

atgcttccta cattctccct ctctacgctg ctattctgca aaatttacag ggcgatctat 1800

gcgataccca atattcgtgg tggttcggaa tacggcgagt catggcaccg ggagggtatg 1860

ttagacaaga agcagaacgt gtttgacgac ttcaacgctg caaccgaatg gcttattgcg 1920

aataagtacg caagcaagga tcgcattgcc atccgaggag gatcaaatgg aggtgttttg 1980

accactgcat gtgcaaatca agcaccggga ctctaccgtt gtgtgatcac aattgagggt 2040

ataattgata tgcttagatt tcctaagttt acatttggcg ctagctggcg ttctgaatat 2100

ggcgatcctg aggatcctga agatttcgac ttcatattca aatactcccc ctatcacaac 2160

atccctcccc cgggagacac tattatgcca gccatgctat tctttactgc ggcgtatgat 2220

gatcgtgtct cgcctttgca cacgttcaag cacgttgcgg cattgcaaca caactttccc 2280

aaaggcccga atccatgcct aatgcgcatc gacttgaatt cgggacactt tgctggcaag 2340

agtacgcagg agatgttgga agaaacggca gatgaataca gcttcatcgg gaagtctatg 2400

ggcctcacta tgcagactca aggttcagta gattcaagcc gctggtcctg cgtaacagtt 2460

tga 2463

<210> 8
<211> 2066
<212> DNA
<213> Omphalotus olearius

<220>
<221> ophM1
<222> (1)..(2066)
<223> chromsomal DNA

<400> 8
atgtcatatc cgctgacctc tgacttaccg cccttcgtga agtattatgg aaaggctttc 60

aaagttccta tgttagctgg ctggcaaatt cttgtcacag gaccgcgtat gatggaggaa 120

attcgaatgg ctcccgagct ttctacccgt gaatccctct tcagagaggt acgctttcat 180

tttcaatact ttgtgtgtgg agcgttcgcc aacgaatctc tttagtgcct acagatggac 240

tataccttcg gccctgaaca tctcattaac ccatatcatt ttcaatttgt acaaatctct 300

ctcggacgag atattggtct tcgaataccc gcgctacaag aggaaattgt aacgttattt 360

tcggaactca ttcctcaaac gaaaggtgcg cagtcgtagt tgaggctcgt gagcttcccc 420

gttatgtact agactattca cgaacattcc ctagattgga ttagactccc gggagcttct 480

actgggcagc aaatcgcgtt cagaacaacg actaggcttc ttgttgggct tccactatgt 540

acggaacgat tcttatgcgc ctctttatgt atcactgagg ctaatacctt cctcttgggc 600

caggccggga ccaagactac aaggcactca atgatcaatg gcctgattct ctggccaagg 660

ctgcgatcac catcagcgtt gttcctcagt ttctgaaatc gtaagacgct cttataacgt 720

tgtagaactt tcgatatgta tctgacctct cacgatttag gttcgtttct cgttctcttc 780

ccttttaccg atcagtgtta gatcgcgcaa tgaaccatgt gcgtcctttg ttggaggagc 840

gctttgaaaa gcaaggacca ttcggcgatg atgcaccgga taaatcggtg cgtcttgtac 900

ccgtaggctc actgtcttac taacggaagt attcattcac gctcaggatg accttcttac 960

ctggttaatg aagaaagcgc caacaaacga gaagtcactc gaagccatta cgaggcgcct 1020

tatggcaaca cacttcgtga cttatcgtcc gctatcactc gtatgtactt cataccacgg 1080

aattcgttga agaattgacc tactattaac tttcaacagg ctacaacgga agtgctgtat 1140

gatctagcag cgcatccatc ttacgtccct gagctacgag aagaagttga agctctcgtg 1200

cgcgagcacg gtgttacgaa ggaaggtttg gataaaatgt ataagcttga tagcttcgtt 1260

aaggaatgtc tgcgcctcca ttcgagccca aagagtaagt agatctctta atttcattca 1320

cacgaattcg tctcaactca ttgtttctct ttcagtcacc atgtcacgta aagttatgaa 1380

agacttcacc ttttccgatg ggaccaccat tccagcagga aatacgattg ctgttgcagg 1440

ctatgccatt catcacgatg aggtatgtcg tacatttatg cgacttgata tcgttttgac 1500

ttttgggcaa tagagaagct ttcctaatcc cgaggaattc gatgggttca gatattttag 1560

gaaacaggac gaagaacagg aaccgggaaa acatcagctg gttactctcg ctcacgacta 1620

tgtcctcttt ggaggtggta gacacgcctg gtaagtagtg aatcgtactg tatttattcc 1680

taggtggtga cataacattt tacagtccgg gacgcttttt ggtcgctgcg atactcaaga 1740

cgatgctcgt gcatgtacta tttacgtacg atgtgaagct ggaggaagaa ggtgtacgcc 1800

ccccagatga atggcctggg atgttggttg cagtacctaa ccaaactgca ggagttttgg 1860

tgagaagacg agaattatct tcttgggaat gaataccgtg tgtctgtgta tgtgtatcct 1920

gtattatgaa ggagaagaat cgcagtgtat ttgctttcaa tgagtaccct cgtataacgc 1980

atgagacggt catcgacgtt aagtacgttg tgatgcggag atatgtagcg ccttcggacc 2040

agtcttgtga aagttcagaa gtttga 2066

<210> 9
<211> 1864
<212> DNA
<213> Omphalotus oelarius

<220>
<221>  ophM2
<222>  (1)..(1864)
<223>  chromosomal DNA

<400>  9
atggccgacg agtgggacga attgttatct tggctcgtat cgagacttca atttgacctc      60

tatgtccagt acagcgcgaa tagagcactt ggaagcttcg ttctgctgct cggaatcgct     120

atccttgtcc ttttcacgaa gaaaatcaaa gagatataca tcgacttcct tgccattgca     180

tatggttacg acttgatgag atcccgcttc aaatacggcg agaacgtttt tcatttccat     240

ctctttggtg tacgtatctc cagtcttacc tttcgtcgtt tatatactca gtgttgacag     300

aatctcgtca tcggattacg aggtcagaaa gcacggagag tgttgttcga agagaagaac     360

ttcagtagta gtgatggata caagatgttc agcaaagtgg tccctgtatg tgaagctttc     420

tacttaccag ttaccattgg gaactttcgt gtagattcct gaggtcactt ttgccccgga     480

acaagaagat tacaccttgg cattcaagaa gcacctaggg actctgctgg ggaaggatag     540

acttaatgac agtgagttac cgtatgacac caccatgctc caacttgact tcttccgcca     600

gtaattgcta ccatgtttcg agacatagaa cgtgtcatgg aaacttgggg cgagaagggc     660

gctatggatc cgttcaataa catcaacgac gtatgtctcc tctcctttcc tttaccattc     720

tgtgaacttc acaaactcag atcgcgtttc tgttgaccgt gcatatggtt accagcgacg     780

aattcgtttc ggatctctct gctctgtctc gatttcaaag ccacttttcc agacagctaa     840

gcagtcatac ccccggaacg attttcttcc catggtttta tcggacagct cagcgcgaga     900

gagagaagtc cgtaatggcc ctcttcaata cattaaccga acacatcgag gcacgaaaag     960

ggcgtaccgc accgacacct gaagctgtcg atcttttact ggcccgtgga ctgaatactc    1020

aagagatttt ccagtttatc cttggattct tattcggagg tgcatttaat acaaccaaag    1080

cgagtatgtt cgagctttat tgtgattttt ttctattcat ggtcaataca tacagcgtca    1140

tggattcttc tttatctcgc actccatcct cagtggggct ctactattcg ctccgaaata    1200

gagaccgttc tctctcgcac atcaattgac aactctgaat cgatgtgcag cagactttct    1260

tcaatctctg tcgaagattg ggaaagctca atgcctacac tcgaccttgt catatctgaa    1320

actcttcgat tggtcatgaa tcaaaccgtg cttcggcgca atatgggcga gaagttgaag    1380

atagatggtt taggcgaaat accgagaggc gcatttctgg tctacagtaa tgccgatgct    1440

catttgaacc ccgagctgta tcctgatcct tggacattca acccccagca cttcagtcag    1500

ggtaaggaag atgatgagta tgcgttcttg ggatggggcg cgggtgagcg ttcatttgat    1560

caaaaacttt gattcacaat tcacaatagt gcaatgcttc aacagggagg caccggtgtg    1620

ttggggttaa gattgcaaaa ttggcaatca aggcgattgt ctcgtcattc ataatgactt    1680

atgagcacga tgtcatcagc tcaaatcgca aagtcacctc gaaaactccc attcccgatt    1740

ataacaagat aaacaatgtg agcaagttac aaacttggaa tttctcgtgc gagactaatt 1800

ctggaatcag ggacacccga agaatggagt gtattccatc agctatagta ggcgcgtagg 1860

atga 1864

<210> 10
<211> 1497
<212> DNA
<213> Omphalotus olearius

<220>
<221> ophM2_cDNA
<222> (1)..(1497)

<400> 10
atggccgacg agtgggacga attgttatct tggctcgtat cgagacttca atttgacctc 60

tatgtccagt acagcgcgaa tagagcactt ggaagcttcg ttctgctgct cggaatcgct 120

atccttgtcc ttttcacgaa gaaaatcaaa gagatataca tcgacttcct tgccattgca 180

tatggttacg acttgatgag atcccgcttc aaatacggcg agaacgtttt tcatttccat 240

ctctttggta atctcgtcat cggattacga ggtcagaaag cacggagagt gttgttcgaa 300

gagaagaact tcagtagtag tgatggatac aagatgttca gcaaagtggt ccctattcct 360

gaggtcactt ttgccccgga acaagaagat tacaccttgg cattcaagaa gcacctaggg 420

actctgctgg ggaaggatag acttaatgac ataattgcta ccatgtttcg agacatagaa 480

cgtgtcatgg aaacttgggg cgagaagggc gctatggatc cgttcaataa catcaacgac 540

atcgcgtttc tgttgaccgt gcatatggtt accagcgacg aattcgtttc ggatctctct 600

gctctgtctc gatttcaaag ccacttttcc agacagctaa gcagtcatac ccccggaacg 660

attttcttcc catggtttta tcggacagct cagcgcgaga gagagaagtc cgtaatggcc 720

ctcttcaata cattaaccga acacatcgag gcacgaaaag ggcgtaccgc accgacacct 780

gaagctgtcg atcttttact ggcccgtgga ctgaatactc aagagatttt ccagtttatc 840

cttggattct tattcggagg tgcatttaat acaaccaaag cgacgtcatg gattcttctt 900

tatctcgcac tccatcctca gtggggctct actattcgct ccgaaataga gaccgttctc 960

tctcgcacat caattgacaa ctctgaatcg atgtgcagca gactttcttc aatctctgtc 1020

gaagattggg aaagctcaat gcctacactc gaccttgtca tatctgaaac tcttcgattg 1080

gtcatgaatc aaaccgtgct tcggcgcaat atgggcgaga agttgaagat agatggttta 1140

ggcgaaatac cgagaggcgc atttctggtc tacagtaatg ccgatgctca tttgaacccc 1200

gagctgtatc ctgatccttg gacattcaac ccccagcact tcagtcaggg taaggaagat 1260

gatgagtatg cgttcttggg atggggcgcg gggaggcacc ggtgtgttgg ggttaagatt 1320

gcaaaattgg caatcaaggc gattgtctcg tcattcataa tgacttatga gcacgatgtc 1380

atcagctcaa atcgcaaagt cacctcgaaa actcccattc ccgattataa caagataaac 1440

aatggacacc cgaagaatgg agtgtattcc atcagctata gtaggcgcgt aggatga 1497

<210> 11
<211> 1234
<212> DNA
<213> Omphalotus olearius

<220>
<221> ophA
<222> (1)..(1234)
<223> chromosomal DNA

<400> 11
   atgagttata gtcgaatcct tgtccttgct gctgtgtcca aatgcatatc gacatttgga 60
   atcgtgaccc cgccttcccc gcaccgatgg atgcttttcg tgcctatcgt cgtaatcaat 120
   gcttactgtt acttcagcac tttcaccgac gataccatgc gtaggaacgc cgtcatagaa 180
   actttggttg cttcggattt cttggtcctc acggacgttc agcgcgaatt ccgtcaggtt 240
   ggacaacggg aacctatctc gaaactcggg ctttgggctc gattacaatg ggctctccgg 300
   ctactactct cccctcgagg tgtagggtgg acgtatgaac ccacatcagc tcttccttct 360
   cgccacaacc ttccgcggcg cacgttcctt cggactcagc tcattcttct tgtatgggct 420
   atcattggaa acgatcttgc ctacattctt accttcaagg atccaggctt cgccaaacat 480
   gcacgtccgt ttagcgagca gcctctcatc tggcgtttct gggcaactgc cgtcttcttt 540
   ttcaaaatca gaaagtgtac cgaagtttat tacttcttcg tagccttcgt acttgtcggg 600
   gtcaacatgt cagaaccaag ttcttgggtg gaattttttg gaagttggtc ggatgcatat 660
   actgttcgtc gttggtgggg gtacgatatc tcacttttga cttggcgcgt ttgatcgtct 720
   tgacgagatt gtttctctgt agaagagtat ggcatcaaag cctgcgcagg gtctgtagac 780
   attttggcgg agacgtatat cattctgact actcatttag atgtttgctt cgcacgggaa 840
   gtacctagct catcgtgtct tcggttttcg cccaggatgc ccagcgtctt cgtacactca 900
   actctatgtc gcctttttct tatcggggat cattcatgtg acgtcgtacg atccacgtcc 960
   gatgtggtat ttcatgtctc aggcagtcgc gatcaccttt gaggaaatcg ttctcaccat 1020
   agcgaagaaa gctggcatga aagctcgaag gggtactcgt ctgctcggtt atgtttgggt 1080
   gtactgcaac ctggtgttca ctttgggccc actcttcgat tccatgaatg cagtcggact 1140
   tggtttggga gggtacgatg ggacaatctt gctaggtcgt tatcgtgact cggagtggat 1200
   gacggggttg ctgtctcatg cgactgcaaa ttga 1234

<210> 12

<211>  1044
<212>  DNA
<213>  Omphalotus olearius

<220>
<221>  ophA_cDNA
<222>  (1)..(1044)

<400>  12
atgagttata gtcgaatcct tgtccttgct gctgtgtcca aatgcatatc gacatttgga      60

atcgtgaccc cgccttcccc gcaccgatgg atgcttttcg tgcctatcgt cgtaatcaat     120

gcttactgtt acttcagcac tttcaccgac gataccatgc gtaggaacgc cgtcatagaa     180

actttggttg cttcggattt cttggtcctc acggacgttc agcgcgaatt ccgtcaggtt     240

ggacaacggg aacctatctc gaaactcggg ctttgggctc gattacaatg ggctctccgg     300

ctactactct cccctcgagg tgtagggtgg acgtatgaac ccacatcagc tcttccttct     360

cgccacaacc ttccgcggcg cacgttcctt cggactcagc tcattcttct tgtatgggct     420

atcattggaa acgatcttgc ctacattctt accttcaagg atccaggctt cgccaaacat     480

gcacgtccgt ttagcgagca gcctctcatc tggcgtttct gggcaactgc cgtcttcttt     540

ttcaaaatca gaaagtgtac cgaagtttat tacttcttcg tagccttcgt acttgtcggg     600

gtcaacatgt cagaaccaag ttcttgggtg gaattttttg gagaagagta tggcatcaaa     660

gcctgcgcag ggtctgtaga cattttggcg gagacgtata tcattctgac tactcattta     720

gatgtttgct tcgcacggga agtacctagc tcatcgtgtc ttcggttttc gcccaggatg     780

cccagcgtct tcgcagtcgc gatcaccttt gaggaaatcg ttctcaccat agcgaagaaa     840

gctggcatga aaagctcgaa gggtactcgt ctgctcggtt atgtttgggt gtactgcaac     900

ctggtgttca ctttgggccc actcttcgat tccatgaatg cagtcggact tggtttggga     960

gggtacgatg ggacaatctt gctaggtcgt tatcgtgact cggagtggat gacggggttg    1020

ctgtctcatg cgactgcaaa ttga                                           1044

<210>  13
<211>  35339
<212>  DNA
<213>  Omphalotus olearius

<220>
<221>  omphalotin cluster
<222>  (1)..(35339)

<400>  13
gtcggagaac gtgaagtctt tcaaaacctt tcgaggcata cttgctacat tccgctccgt      60

caattagtgg aacaatatta ttcggtaaat aatctctagg acaatggtac tcacattttc     120

caccccctct caggcgcagg gattccttca gaaaactatc aagcttgtac atcttattga     180

tcccctcctt cgtgatacct tcttctttaa ctatcggctc tacctctgct cgcagttccg 240

gcacatatga tggatatgtc gccaagtcga atagcgcttg cgtgagtgcc ttgtatccat 300

cagtccctgt caaaattctg acttcgcata gatgtgccca ccatagacgc aggtcggtac 360

gctgcaaaca tcgttgccag tagcctgata gtaattgccc gaacggattt gtcgctctct 420

ggtgctttct ccatcaacca agtgagtaga tcattctaga actcgcacac atgcttagat 480

ataaaggatc agggtttaca cttggaagtc taataccaac cggctttcct ggccaatctt 540

ttccgagcgt tctctccttt tcaagtcgtt cttcgattac tggagcaacg tgtttcattg 600

cacgcttcaa agtcttagga atataaaagt ataaagcttt ggcgacgatt ctgaatagca 660

tatagcgtga gtgtgacgag tgcggttacc ccaagagacg tgtggaaacg cacggtttga 720

aaaggtcagg tgtgagacca acgaacatgg cggtttttac gagtgcgtcc gtatacgtat 780

cgttgatatc gttgtagtcc ggattatgac ctataaatta cttcataggc agagatcatt 840

ccgataacag aggttgaacg cacacaatgg aggtcctacg aaaagtctat tggtgacccg 900

gagaaccatg gacttgcccg taagccatgc gggaacgcta gcccaatcta gtatgataag 960

taggataatt tttcatccat ttgagatctt cttaccgtca gtaggaggaa ctaattcaga 1020

gaaagctgtt atcacttctt cttgcatttc aggaaatcgg ccacctaggt cgcgcgtgag 1080

ggaaaggtgc acaaaacgaa agtgataagg gttctcgagt atatggggct ccatcgtgta 1140

gtcggtttgc atgccctaat aagaacgatt caaatgtgaa ttagagcaag aatgagaatt 1200

gagtacctcc ttgatagaac catataaaga taactcatcg tcgctggctt tccggacatc 1260

ctcaaggaga tcgttaccgg taaccacgaa agtcatcatg aacgcagtcg ggattgtgaa 1320

caacttgccg tagaactacc attcgaggat tgagtgggta tcattagatc atctttttcg 1380

catacatact ttgtcgtagc cctcctgaat gaaatcgcga gtaccaagca gaagttgaaa 1440

agttccatac aaggtcccaa tgacgcctgt cggtcctaca cgtataacgt gtctatacta 1500

gagagatgcc ttatcgtctg agaattggga actagacttg aacgcaaacg accgtacctt 1560

gaagctagtt ttcctctcat cgataaaaca tcgagttatg atcaaagcaa cgacggatac 1620

accaaatgca agcaatatgg acgtggatat cattgctagg agtgccacct ggatgctact 1680

gggtataacg cacttattca gtggcggaag gcaccctcaa ggatgtttaa tctccgaccc 1740

atctgtactt gaaatttcgg cgaacgccga gagaccaaat actgcaaagc tcccgaccct 1800

ggtccacaag agagacatct cgcttggaag caggactcca tcatgttttt cctgtcgtgc 1860

gtcgtgcaac acgacatacc cggatcatta ttaaaaatgt gattcggtat gtctgatagt 1920

cttttccatt tgtgacattg tcagtggaga tctaggaatt tagatctgcc tggttttctt 1980

gaatttttgt attttcgtcg gattttcttt caagctcagc cgtcacagct ttccaagtat 2040

cacggcgtct tttcgacttc agtaccttga gggttcctgc aacctggtgg cccggaagca 2100
actgagtcga ctcgccgcca acctcacgaa tatacacata cgatgttctt caccttttca 2160
acatcttaca tcgctctggt cgtcgccgtt cttgtcgttg cccgctcaat cgttaccttg 2220
cgtgctaaac acaaggtttc gtattcattc atgttctatc ctcgagctaa attgggttga 2280
cataccccac agctcgatct gcccaccatc ggaggggcag gactcgtcca gtcttatgtc 2340
gctgcataca aatttctctt cggatcccgc gatctcattc aagaaggtta tgaaaaggtc 2400
gtctagcgat tccatgtcat atccgctgac ctctgactta ccgcccttcg tgaagtatta 2460
tggaaaggct ttcaaagttc ctatgttagc tggctggcaa attcttgtca caggaccgcg 2520
tatgatggag gaaattcgaa tggctcccga gctttctacc cgtgaatccc tcttcagaga 2580
ggtacgcttt cattttcaat actttgtgtg tggagcgttc gccaacgaat ctctttagtg 2640
cctacagatg gactatacct tcggccctga acatctcatt aacccatatc attttcaatt 2700
tgtacaaatc tctctcggac gagatattgg tcttcgaata cccgcgctac aagaggaaat 2760
tgtaacgtta ttttcggaac tcattcctca aacgaaaggt gcgcagtcgt agttgaggct 2820
cgtgagcttc cccgttatgt actagactat tcacgaacat tccctagatt ggattagact 2880
cccgggagct tctactgggc agcaaatcgc gttcagaaca acgactaggc ttcttgttgg 2940
gcttccacta tgtacggaac gattcttatg cgcctcttta tgtatcactg aggctaatac 3000
cttcctcttg ggccaggccg ggaccaagac tacaaggcac tcaatgatca atggcctgat 3060
tctctggcca aggctgcgat caccatcagc gttgttcctc agtttctgaa atcgtaagac 3120
gctcttataa cgttgtagaa ctttcgatat gtatctgacc tctcacgatt taggttcgtt 3180
tctcgttctc ttccctttta ccgatcagtg ttagatcgcg caatgaacca tgtgcgtcct 3240
ttgttggagg agcgctttga aaagcaagga ccattcggcg atgatgcacc ggataaatcg 3300
gtgcgtcttg tacccgtagg ctcactgtct tactaacgga agtattcatt cacgctcagg 3360
atgaccttct tacctggtta atgaagaaag cgccaacaaa cgagaagtca ctcgaagcca 3420
ttacgaggcg ccttatggca acacacttcg tgacttatcg tccgctatca ctcgtatgta 3480
cttcatacca cggaattcgt tgaagaattg acctactatt aactttcaac aggctacaac 3540
ggaagtgctg tatgatctag cagcgcatcc atcttacgtc cctgagctac gagaagaagt 3600
tgaagctctc gtgcgcgagc acggtgttac gaaggaaggt ttggataaaa tgtataagct 3660
tgatagcttc gttaaggaat gtctgcgcct ccattcgagc ccaaagagta agtagatctc 3720
ttaatttcat tcacacgaat tcgtctcaac tcattgtttc tctttcagtc accatgtcac 3780
gtaaagttat gaaagacttc accttttccg atgggaccac cattccagca ggaaatacga 3840
ttgctgttgc aggctatgcc attcatcacg atgaggtatg tcgtacattt atgcgacttg 3900
atatcgtttt gacttttggg caatagagaa gctttcctaa tcccgaggaa ttcgatgggt 3960

tcagatattt taggaaacag gacgaagaac aggaaccggg aaaacatcag ctggttactc 4020

tcgctcacga ctatgtcctc tttggaggtg gtagacacgc ctggtaagta gtgaatcgta 4080

ctgtatttat tcctaggtgg tgacataaca ttttacagtc cgggacgctt tttggtcgct 4140

gcgatactca agacgatgct cgtgcatgta ctatttacgt acgatgtgaa gctggaggaa 4200

gaaggtgtac gccccccaga tgaatggcct gggatgttgg ttgcagtacc taaccaaact 4260

gcaggagttt tggtgagaag acgagaatta tcttcttggg aatgaatacc gtgtgtctgt 4320

gtatgtgtat cctgtattat gaaggagaag aatcgcagtg tatttgcttt caatgagtac 4380

cctcgtataa cgcatgagac ggtcatcgac gttaagtacg ttgtgatgcg gagatatgta 4440

gcgccttcgg accagtcttg tgaaagttca gaagtttgaa tatcaagtat atcactggca 4500

taaacataaa aggaagtgat gtccttttcc cgctcagacc aacggaacaa cattgtagca 4560

atatgcatgt gctccttagc cgtgactcag tacccaatcg aaaacattca caacgcgatc 4620

tgacatatgg attgtaattg tacaaagtat acaacatgag ggtttacgat ttaaaacacg 4680

ctcctacact gctccaccga ctctcaaaac gactgaatca gatggaaaac gatttcactt 4740

ggaagttggg atcagtctcg aactttgcat cgctgatctg gtcgccattg aattcgtaga 4800

ccgccttgaa cggaacctcc ctgaagaaca gacagtcagt accgatactc atctgagctg 4860

cagatcatat agcgcactcg actttgagct tgaccaagga tttgccctgt tgagacatag 4920

tcagacagga tctggaagtc gaaagatagg tattgatact taccacgact acaacctttc 4980

catctacttc tttgatatcg ctgatgacag tctatgtaag ccgtcgcgat gacatgagct 5040

ctagttatca atcaagaaag ggagaagacc acttgccttg taatctaaga ccttcagcag 5100

ctcattgtga gcagtgagcg catcagtctt gcggaactgc acagacgtgg gtcccccaag 5160

gaaggacgcc gccaacgaag tgtggcttga gctcaaatcg tcagccacat gagcgccagc 5220

atgcacatgc ttgccgtcgt tgagaaggtc aaagtagtta cgaacaattt gaactttgtc 5280

agacatgatg tggttgagct tgtgtggtat taagcaacct gaagtcgggc tgaaatggat 5340

ttatagcaga ttttatagca gcatctactt ttcatgtgac atggatgggc tgtgagatat 5400

tcgacggggg gctgcaacga gataagagga cgtacagatg gggtagcctt gtcatttcca 5460

ccggaactag acctatcacc ctgtcctcac cgaattgtcg gaatctccaa aatcacagaa 5520

tattctaaca tgaaacgtaa cctgatgcct atactaagcg tgtcagaatt gcaaggagcc 5580

aattgaagaa cattcaggct gtcgaaatat ggtgccccga tgtattgagg ttattctcgc 5640

aaaggccgtc tttcactcta gacatatata tgttatctaa gattgaatac atgtgatgga 5700

ttctgacctg gttctctgcg tagactccgc tggtgccact ccgactcgat gaaaatcttt 5760

ccagcaaaca ctcctgctca agccttcttc aagccatgag gtgtttgttc agtggcaaag 5820

atacgaaatg agaaaaaaaa tatttgcaaa atgtgagctc tcgttcacag cctgcgccag 5880

ctggcaccgg gcgcgtaagt gacggtatgt gagaaagcaa atcgatcgat cacatgatcg 5940

tgttttcttg aaaatgcgga ctgcgcagtg cgatcaacat gccttttact tacagtgacc 6000

agtacacaaa taagggtaca atagctgttc aaataggaat ggaagttaaa tagagaacgg 6060

aatgttctga tagtttccct attcgacata cacgtaggat tttaaaaccg cctaagtgac 6120

acctgatacc acacccgacg agaggagaga ttgaaatcgg agaacatgag ccaattcaag 6180

tgtatcagga ccaagctaaa tgttttagtt tggaatgtta tggaactgtc acaggaatac 6240

tgagatgtaa aaaatacata caactcgtta catcattaca tcgactacct tcctgataac 6300

cacctgtcac gtaaaagtcc caatactgac ctgcctgtat agtcattgaa ttcaacaacg 6360

ttcatgatat taaaccacgg ccccaatata agaacgaggg cgcaatgtag cccaacactc 6420

tgtaacgggc aagagtatgt tgataatgct tgacagcaag aatgcgacgt aaatctgacg 6480

ggtgtatgag gacgcattgc tgccgtgagg gaaacaaaag acgggggtgg cgaggaactt 6540

cccgcgtatt gagaagatct agactcttat ggttaggagc acgttccttt gagcaatgga 6600

ataagttctt actagatctt actcttcgcc gctgctggtt gccacattct cccaaaagat 6660

atcgtcagga ttgctaatcg aagatttacg gatccggatg ccccacttac agaatcgacg 6720

tatggcgtat gtatcacccc attttccaaa gaattctggc catgaattgg gtttcgacat 6780

tccaattccc acaaatatca tcgagcaaca tttgagcgcc tgctcctggc cccaaagcta 6840

gataccgatt tttgttgtcc agcaagattg ttggcaagcg actattggta cttgagctag 6900

gccagttctt ctacttcaat aaacttggaa tggatattgc agactgtcgc cccgcgaagt 6960

cacccaagta atgcttagta tgatccgaga catcgttcga gtcaccgtat gcgaccttga 7020

tatcatcggc gccagcatgc cctccgttcc tgggccttct tcaagaacta aagaagatca 7080

tagtattagt aagtactcgg catcaaattg ccaattacgg tatatactta taacatgcgt 7140

gagaacttct gagaagcaaa attattcccc ggcggttctc acgaggattt actgtgttga 7200

taaccgacta tcactaccgg aacctgccga ttggggaaca aaaccaattg gatatctcac 7260

tttgaccagt tgtatagatg gactacatac tcgtgtacaa cgtcgcggaa gactacaagt 7320

ttggagtgca ggttgaactc cgtatgaacc tttcaaacat gtttctcaag ataaggtcaa 7380

attcttgaag cctagctgac attagttgtg cgcttgaatc gcaaaaagca accctcattc 7440

tttggtttac cctgaattcg aacatccgac acagctccag gaacgacagt aatgataaat 7500

caacttgaag caaacctacc aagactagga tgtcgttata catagtccgg catcggaacc 7560

gcctcagggt tgaagacttc gtggtcatga acagtgcgac aattgacttg atctccaact 7620

tggcaagatt tgataccaaa caagcggtgt tatgcgatgc tgaattagca agtaattgta 7680

gcaacaagct gccccgagtc ctaaaaatgc ggacgggttt tcctctctgt caggtctgaa 7740

tcattccgca tcaaacgaat gaggacttga gagtataagg ccggatcgag atgtgtgtct 7800

gcaagagtgt aagccagaaa tttttaatct tcaaagaaag ccttgcgagc ctcttgcatt 7860

gatggcagtg ataggaagct atgaatacaa tatttcagct gccccaaatc tgccacactt 7920

acgttgatgc attgcgagcg ctcagtcgta atccggaggt gacgatcgta tgcgcggaat 7980

agaaagagcc tgaggtctgt tgtactttac tcttgacgag gatggcaaag ctgtgacaag 8040

gaccagagcc tcaggactcg gagtagtgta agccacaccg ttgaacttac cttctccgca 8100

tatctcacac caaagacatt acaggacatt tattaagaga caccgtagtc cgtatatctg 8160

gtctttgaga tgcaccaaat ctctggcgga gaaaagacct cccaggaaag ggctaatacc 8220

tttcttccta ccgccgatga ttagacacca gtgaacattc ctacgcaaaa ttcgactaca 8280

actcacgctg tcagaagatc tcattctatc atgatttgga gcttccttag cttccatctc 8340

cacaggatga aagatgaaag atgggttttg tattttgaag tcacgttcgt tcttttgttg 8400

gcatgcacga tggaaccagc actggcagca gctcaaagtc ctttgcgagg ttttgaaggt 8460

cagctccgta aacagtttac atatcagtct catccaatat gtacgaacta cagagtatag 8520

ttacaatttt cagcttatct tacgacagtg aagttaattt ttctcttacc tcgtctgtat 8580

tcaattgaaa cgaaaagccg aaaacaagtt gaactcaacg tcacgcgtca acccgcgctc 8640

agcttatcaa atgtcctctc acgaagactc tgacccactc ctctcgccag aatctttatc 8700

attgatcaac actgattact gctgagcaga cttgggtgtc gtgaaaagga ttagtgcggc 8760

attgatgata gacgtcgata aacctccgat gttatcgtta ctagatgtcc agctatctca 8820

ataggcggta gtggcaaaaa ataggtgaca taagtgtccc tagccttgta catcgcatta 8880

cttgtaccgt caactcttga acttactgat ccttacacag gtgaccatcg gagctcaaat 8940

ggtatgatga gaatagatac cgactgccaa tctgtagaat ttgaagaagg taagtaggtg 9000

aggaacgccg ttggcatgat gattatcttc aatgcccatc tgtgctaagc gcagattcgt 9060

atcgcaaaag aagggtacta gtccttttga tacaagagca ttctcattcc gacgacatga 9120

ataattgacg cggaggaaat agcacccttc tgatggatat ttgaagacag tgaacaagga 9180

aggaggatca ctcactccgt tcttcatttt ttatttttat tttcctttgc ggaggctggc 9240

gtgtgaagct cgaatgaaaa aggaccttcg tttctttgct ccaagaccgg actaacaggt 9300

ctgcacatat ctagaacaat cagttggaaa atcattttct cgagcaagac gtctgtctcg 9360

gaaaacatac catgcgatat tatgttctaa gtactttcct aatgtgaaga tggctacatt 9420

cgtatgtgtt cactgcgttt actgactttg gacaaggcca tggacacaaa tcactgctga 9480

aatataaagg agctgaaaat ccaagatatt cttcatcaat atcgctgatc atatcatgga 9540

gacttccact cagaccaaag ctggctcact caccatcgtc ggtaccggta tcgagagtat 9600

cggacaaatg acgcttcagg cgttgtccta catcgaagcc gccgcgaagg ttttctactg 9660
cgtcatcgac cccgccactg aggcattcat cctcaccaag aacaagaact gcgttgactt 9720
gtatcagtat tacgacaatg gcaagtccag attgaacact tacacccaaa tgtcagaggt 9780
aagcttcgta caccctcaac agtcgcccac accccgacgc tgacacacgc atagctcatg 9840
gtcagggaag tccgcaaggg cctcgatgtc gtgggcgtct tctacggcca cccaggagtg 9900
ttcgtgaacc cgtctcaccg agctctggct atcgccaaga gtgaaggcta ccgagcgagg 9960
atgcttccag gcgtgtctgc ggaagattgt ctcttcgcgg acttgtgtat cgatccttcg 10020
aacccgggtt gcctgaccta cgaggcatcg gatttcctga tcagggatcg cccggtcagc 10080
atccacagtc acttggtcct gttccaagtc ggatgcgtcg gtattgctga ctttaacttc 10140
actggattcg acgtaagttc tttcactcca atagctcaag atcttccgct tacctcctca 10200
cgaatggcca gaacaacaaa ttcggcgttc tcgtcgaccg tctcgagcaa gaatacggcg 10260
ccgagcaccc tgtcgtccat tacatcgcag ctatgatgcc acaccaagac ccagtcaccg 10320
ataagtacac cgtcgcgcag ctccgtgagc ccgagatcgc gaagcgtgtt ggcggtgtct 10380
cgactttcta cattcctccc aaggccagga aagcatcgaa cttggacatc ataaggcgcc 10440
tagagctctt gcctgctggg caagttcccg acaagaaagc gcgtatttac ccggccaacc 10500
agtgggagcc cgatgttccc gaagtcgaac cctacagacc atctgaccag gctgccatcg 10560
ctcagttggc tgaccacgct cctcctgagc aatatcaacc tctcgctact tcgaaagcca 10620
tgtctgatgt tatgacgaag ttggctttgg atccaaaggc actcgccgac tacaaagctg 10680
atcaccgcgc ctttgctcaa tctgtccccg acttgacgcc tcaggagcgt gcggctttgg 10740
agctcggtga ttcgtgggct attcgttgcg cgatgaagaa tatgccctcg tcgctcttgg 10800
acgctgctcg tgaatccggc gaagaggcat cccaaaacgg tttcccatgg gtcatcgtcg 10860
ttggtgtcat cggtgtcatc ggatcagtca tgagcacgga ataaacggtt tggaatgtca 10920
tgatatcgcc gtccttggta accaattctt tcttctgtta gccaatcaag aacttgtagg 10980
attaatgtgg atgtcgtcag aaaatagttc tatgtacaag tcaagtcggt tatctgtgat 11040
taacaatgta tgaaatttga acctcatcat tacatgaaaa cttgcctttc actctcggaa 11100
acaatgaacg aatgatcttc gttctctgag ctgctgtgat ttcaagcaat gtattgttag 11160
aacataaccg gcaggaacta tcaattgatc acttacttat gtaacaatgt tgcgccggta 11220
gtaggtacat attcgttatc ttgatctgtc aacattatac acaatcagtg ctaaagaaaa 11280
agatcaaaag agaatagatc acattttaca gattctaaga cgagcttgtt tcttcgatgc 11340
tgcgagattc ataccccgat gttccgtacc atatgcacac attacttcct gcgaacacag 11400
agaagaacat tgaaacacaa aaactaccga ctatttccag catttgagcc atccatcatc 11460
cgtagctcgt attcaatttg cagtcgcatg agacagcaac cccgtcatcc actccgagtc 11520

acgataacga cctagcaaga ttgtcccatc gtaccctccc aaaccaagtc cgactgcatt 11580

catggaatcg aagagtgggc ccaaagtgaa caccaggttg cagtacaccc aaacataacc 11640

gagcagacga gtacccttcg agcttttcat gccagctttc ttcgctatgg tgagaacgat 11700

ttcctcaaag gtgatcgcga ctgcctgaga catgaaatac cacatcggac gtggatcgta 11760

cgacgtcaca tgaatgatcc ccgataagaa aaaggcgaca tagagttgag tgtacgaaga 11820

cgctgggcat cctgggcgaa aaccgaagac acgatgagct aggtacttcc cgtgcgaagc 11880

aaacatctaa atgagtagtc agaatgatat acgtctccgc caaaatgtct acagaccctg 11940

cgcaggcttt gatgccatac tcttctacag agaaacaatc tcgtcaagac gatcaaacgc 12000

gccaagtcaa aagtgagata tcgtaccccc accaacgacg aacagtatat gcatccgacc 12060

aacttccaaa aaattccacc caagaacttg gttctgacat gttgaccccg acaagtacga 12120

aggctacgaa gaagtaataa acttcggtac actttctgat tttgaaaaag aagacggcag 12180

ttgcccagaa acgccagatg agaggctgct cgctaaacgg acgtgcatgt ttggcgaagc 12240

ctggatcctt gaaggtaaga atgtaggcaa gatcgtttcc aatgatagcc catacaagaa 12300

gaatgagctg agtccgaagg aacgtgcgcc gcggaaggtt gtggcgagaa ggaagagctg 12360

atgtgggttc atacgtccac cctacacctc gaggggagag tagtagccgg agagcccatt 12420

gtaatcgagc ccaaagcccg agtttcgaga taggttcccg ttgtccaacc tgacggaatt 12480

cgcgctgaac gtccgtgagg accaagaaat ccgaagcaac caaagtttct atgacggcgt 12540

tcctacgcat ggtatcgtcg gtgaaagtgc tgaagtaaca gtaagcattg attacgacga 12600

taggcacgaa aagcatccat cggtgcgggg aaggcggggt cacgattcca aatgtcgata 12660

tgcatttgga cacagcagca aggacaagga ttcgactata actcataact cgtcgatgac 12720

tagagacgca acgacgaacg tagagcgctg aggtctacta tggagctcct ttagtgcttg 12780

agtaccatca agatagtcat atgtttgact cacaccccgt tttccagatt ctactggttc 12840

cgcaacgtat caatgtatta agcccgtgac agacggcatg ttaaggatca aggtcctgac 12900

tcctgcaagc cccaaacgtt caaacttcca agcgaagtgt cattcatcgg cagaccctgg 12960

gtcgtgttcg catattggca aagcaaagtc gatggaagaa attaaaaaat tagcaagcag 13020

gtgacggtca aaagaatgga cgaaaagaat ttttcatccg tgaattactt acataagtga 13080

tgtggttaca taatcaacat ttcagattgg cgctgctctc cttctgctct ccttctgctc 13140

accggaacct ttgtcatcgg catccttaac atcgtcgaag caatggccga cgagtgggac 13200

gaattgttat cttggctcgt atcgagactt caatttgacc tctatgtcca gtacagcgcg 13260

aatagagcac ttggaagctt cgttctgctg ctcggaatcg ctatccttgt ccttttcacg 13320

aagaaaatca aagagatata catcgacttc cttgccattg catatggtta cgacttgatg 13380

agatcccgct tcaaatacgg cgagaacgtt tttcatttcc atctctttgg tgtacgtatc 13440

tccagtctta cctttcgtcg tttatatact cagtgttgac agaatctcgt catcggatta 13500

cgaggtcaga aagcacggag agtgttgttc gaagagaaga acttcagtag tagtgatgga 13560

tacaagatgt tcagcaaagt ggtccctgta tgtgaagctt tctacttacc agttaccatt 13620

gggaactttc gtgtagattc ctgaggtcac ttttgccccg gaacaagaag attacacctt 13680

ggcattcaag aagcacctag ggactctgct ggggaaggat agacttaatg acagtgagtt 13740

accgtatgac accaccatgc tccaacttga cttcttccgc cagtaattgc taccatgttt 13800

cgagacatag aacgtgtcat ggaaacttgg ggcgagaagg gcgctatgga tccgttcaat 13860

aacatcaacg acgtatgtct cctctccttt cctttaccat tctgtgaact tcacaaactc 13920

agatcgcgtt tctgttgacc gtgcatatgg ttaccagcga cgaattcgtt tcggatctct 13980

ctgctctgtc tcgatttcaa agccactttt ccagacagct aagcagtcat acccccggaa 14040

cgattttctt cccatggttt tatcggacag ctcagcgcga gagagagaag tccgtaatgg 14100

ccctcttcaa tacattaacc gaacacatcg aggcacgaaa agggcgtacc gcaccgacac 14160

ctgaagctgt cgatctttta ctggcccgtg gactgaatac tcaagagatt ttccagttta 14220

tccttggatt cttattcgga ggtgcattta atacaaccaa agcgagtatg ttcgagcttt 14280

attgtgattt ttttctattc atggtcaata catacagcgt catggattct tctttatctc 14340

gcactccatc ctcagtgggg ctctactatt cgctccgaaa tagagaccgt tctctctcgc 14400

acatcaattg acaactctga atcgatgtgc agcagacttt cttcaatctc tgtcgaagat 14460

tgggaaagct caatgcctac actcgacctt gtcatatctg aaactcttcg attggtcatg 14520

aatcaaaccg tgcttcggcg caatatgggc gagaagttga agatagatgg tttaggcgaa 14580

ataccgagag gcgcatttct ggtctacagt aatgccgatg ctcatttgaa ccccgagctg 14640

tatcctgatc cttggacatt caacccccag cacttcagtc agggtaagga agatgatgag 14700

tatgcgttct tgggatgggg cgcgggtgag cgttcatttg atcaaaaact ttgattcaca 14760

attcacaata gtgcaatgct tcaacaggga ggcaccggtg tgttggggtt aagattgcaa 14820

aattggcaat caaggcgatt gtctcgtcat tcataatgac ttatgagcac gatgtcatca 14880

gctcaaatcg caaagtcacc tcgaaaactc ccattcccga ttataacaag ataaacaatg 14940

tgagcaagtt acaaacttgg aatttctcgt gcgagactaa ttctggaatc agggacaccc 15000

gaagaatgga gtgtattcca tcagctatag taggcgcgta ggatgaggac gacgtacatg 15060

acatgagatc tagagtctgt tttactcgat gaggaacaag ccgactgaaa cgttcctttg 15120

agtgtccgac attaccttcg tgaagagcaa cagcggcgga aaaagcttag cttcgactat 15180

gttaaacatc tgacagtata aatgaacgcg gctatcggac tctatcggag tggctaatcg 15240

cgatatgtct gaggaatgag gaatgattgt aagtacagta tgtatgtata tatatttggc 15300

aatctttaat tatcacaact gtggcggaat ttcggggttt tgaaaattca aagagtagct 15360

tatctatgta gcacgaccct gaaatcgtag ttcgccggct agtacagtaa gagctagtca 15420

actacccgga gtgcgtcagg tgcgtcctcg tgctgaatgc cgggacttaa gacggtaggt 15480

cgcagtaggt agcggcaggt acactaggaa catacatatc tagccgaaat gcaccacaga 15540

atctgatgga agggaagcct gcgggatcct gtaacgagtc aaagacaaca tacaactact 15600

gtcagatgta tatcacaaga acgatcgagg gatggctgca gtcctgctgt agtccttatg 15660

tgcaagttat gaggcgaaca acacgcggtc ccgcgagcca gtgcagggtt caaggtcatg 15720

catcatgact aggactgcgc ataatacgta ctgacaatta ggctagtact tgtattgaat 15780

cctgcaggaa ttggagtaca cgtttcgtag accaaccaac caattatggt gtccttttca 15840

gaatatcatt caaagtataa ccctgttacc ctgctagact tcatgtgttc cgtggtttcg 15900

gaggatctcc cgatatcgtt caatcaacac agcaaactcc ttcatcactt cgattgtact 15960

gacgagtcgt cttcaaacgc ttttcaaact gttacgcagg accagcggct tgaatctact 16020

gaaccttgag tctgcatagt gaggcccata gacttcccga tgaagctaag aaagttaaat 16080

acagagagcc cttacatttc gtcgctggac gtacctgtat tcatctgccg tttcttccaa 16140

catctcctgc gtactctaca aggacgataa taagcaaata gtgtaaatac cgccatccaa 16200

taatgcatac cttgccagca aagtgtcccg aattcaagtc gatgcgcatt aggcatggat 16260

tcgggccttt gggaaagttg tgttgcaatg ccgcaacgtg cttgaacgct gcgtaagggt 16320

aagtaaagta tacgattcca tcgaacctct gttgaaccta ctgtgcaaag gcgagacacg 16380

atcatcatac gccgcagtaa agaatagcat ggctggcata atagtgtctc ccgggggagg 16440

gatgttgtga tagggggagt atctagaaag tcctatcttg agtgacgaaa agtcaatata 16500

gtagattgac taacttgaat atgaagtcga aatcttcagg atcctcaggc tacaaccgtt 16560

aggcgaccgt cagaatcatc gcatacttag taagtgtagg tattcgtaca tcgccatatt 16620

cagaacgcca gctagcgcca aatgtaaact taggaaactg gtgacacagg aattagtgag 16680

caagacaatg agcaagttca gaatgtagtc aactgactct aagcatatca attataccct 16740

caattgtgat cacacaacgg tagagtcccg gtgcttgatt tgcacatgca gtggtcaaaa 16800

cacctgcgaa catcggatac tattcagaga tcggaattaa cttcagctag gtaataggtt 16860

tcgcacctcc atttgatcct cctcggatgg caatgcgatc cttgcttgcg tacttattcg 16920

caataagcca ttcggttgca gcgttgaagt cgtcaaacac gttctgcttc ttgtctaaca 16980

taccctggat aacgctcagc aaaacatgta tcatcgatca tcagaccgac atagactcac 17040

ctcccggtgc catgactcgc cgtattccga accaccacta aagtaggtca gcattaggac 17100

agcaatctga acgcttgatg gatcaccgaa tattgggtat cgcatagatc gccctgtaaa 17160

ttttgcagaa tagcagcgta gagagggaga atgtaggaag cattgctagg gagaaaccgc 17220

catagccgta ttgtaggaca ggggaagttc catcgagcag tacatccttc gggcgggtga 17280

taaacatgtg aacactacgt gatttgttag tcctctgtga ttttttgtga agttgtagta 17340

attacgaagt cccgtccttg gatggataga aaacctgatc atggtatttc agtcgcaagc 17400

gcgatgggat ggtataattg acctcaccga ctcagtcata aagtcatcta gattgaggcc 17460

cggaatactt atagcacgaa atagtgaaga atgtcctttc tcttcaccga acgtgtacct 17520

ggtcgggctc tgcatcagtc cgggtaacag aagggttcca cgactactac gtaccgatac 17580

acagttccag gagagaggaa actgctaaag agaacgaaaa tatcgttatc ctgccgtcga 17640

cctgatacca taaactgtcc aagtaaatcc tcaaagatac gcccgagggg tttcccagtg 17700

gttagatcac ggatatgaat ctcgtggcta gcattacgaa gataaacaag taggagcttg 17760

tcttgggcgt gaatgcttgc tgaaacgaga aacgcgttag ggtcttctgc tatgagcgtc 17820

gtcagaccct cgtctgggtg gttggcatca aacgtcgcca ctttgtagtt cttggccttg 17880

agattggtca tgaaatagat ctttgaaccc tcgtctccga ctctgccagt atggtagtaa 17940

gtaattgaat acctagaacc tcagatctta cgaacattgt aaacctagcc tcccatggat 18000

catgaaggtt tttccatttc atattcgtcc caatgtcatt ttcgtgaaga tctgcgatct 18060

tgactaattg agtgtgactt gcgtcgcgct gcacgcatct ttagagaact gatagcggtg 18120

tgaattgaac cagacgcacg ctcgcggcaa agaggatgta tcggccgcta cataaacaac 18180

gaattagcat agcgcgttta tgaagaaaca cagcctacct cggagtgagc tgcaccgacg 18240

agatccagaa cggatgctca ggatcctcac tgaagactct tcaacatttg agatcggcat 18300

cattcgacat agagtactca ctgaacaagg acgtcactct cctgatcctc gccaatccga 18360

tgataataga cctgatagac gcatcaagaa caagataaac tacgatcaaa agaatctcac 18420

catgcaattc attgctggtg cattaccttt tccttcgtca tcctcaggag gatacctaga 18480

atagaaaaaa cctaacgcaa acgccatcta gttcaattcc tcatcttcaa tcgttgagaa 18540

ttgacctttg ctgtcgctag tccaagatac aatgaagaaa cggacgtgtc gaatcttgtc 18600

gttcatccgt ccaggatcct ttcctcgttc ttgtgacgga agatgcggag acgacgtctt 18660

tcgaacgtaa atagacatcc agtcggatcc ctgtgacgtc ctatgagtaa cgtccgaatg 18720

cattatgaga tagatacgaa catgttcaga tacaccatat gcccaataat tcccacatgg 18780

actcatgacg aaaccagtaa gtgcggcgtt gccatcaagg gagagcaagt taggattgaa 18840

aaagagttct ccaccaggac cagactcagt gaggacagta tcctcctccc ccatcctcac 18900

acgatacagg gaaagctgag attgaaggcc atcgttatac tcgaagtagt aatgtccgtc 18960

gctttcaggt ttcaatgcgc taaatctcct gtaattccag ctcttcttca gcgtctcgag 19020

ccatgcttct ctgtaaaagg actgcagtaa ggtcaaagtg ggccagaaaa ctcgaggata 19080

cctaccgatc gggattttca tctagatagg ttcgtgcaaa cttgcgttgt gaatgaacaa 19140

acgcctgttc gtgccattca gaataaggtg taagaattgc ggaatcgttc acgaaccttc 19200

gtttcttcac tatcctcgaa aggtacttca agttgactgt agggatcacg aaccgtaaca 19260

gagccatgca acttgctact gtaagttatt gctgaagttt cgtccctttc aacaggtgga 19320

tatggtcccc atcctggaaa cgacatcgtc ctacaactga gcacaagaag aagatcagcg 19380

acgtcgattg tatccacttc aatctcgagg agcagtgtgt tctcctgaac atcgtgcgat 19440

ctcgttactc cattcggtga taagactaaa ggttccgtca tagcacacga aacccagatc 19500

acgtctgatg agattcgtat taacggaagc agagtgaaag actgagtacc atacttggcc 19560

ccaacatttc caatgccaac gcggatcggt acgaatcaat gagtatatgt agattcacga 19620

ttgtgcagct gacctgctaa gatagatcag ggtctttcgc atgcatcgtc taatgattgc 19680

cgttattctg gaactctaag acgaggagga gaaagtatgt actcaaacaa gtaatcacga 19740

gaaaagggtt tgacattctg acagcgggag tggcgtacag acgatgctgc acggactgtt 19800

acttacttat ccgctgcccc caggtgatgg aaatccccca acaaattgat gtcgttaaga 19860

aagacatcct cttccatcga tctttccagg catcctgtcg acaagtgaac gtactgaaag 19920

aagaaaatca tgagagcctg atataaatca catcgctgag cttcaggaca agaagatgag 19980

atgtaggaga taagaacgca attcatacca acaacagcac agtcccaaag tagatttcac 20040

cgccggagtt gaccggggtg tagacctgaa ccggatttcg acaaatcacc gcaagttggc 20100

ccggttatac aacgccatcg actttgattc acaagccagc gaatatctcc acccgctgca 20160

gcatacctga tgtttgcctt gcctgttgat gttcatgaag ctattattcg cgagctcttt 20220

gccagaaagg acaaggcgac cgtaaagtct ttatcgcttg tatgctcctc cctgacgcat 20280

atctgccaac ggcacctcct ctcgcatctc accatcacct gtcctacagc acaacgcaaa 20340

tacgaacggg gccagcgctc gagtcacgca acgcaggagc tactccaatc atcacctcac 20400

ctcatcccaa tcattcggtc gttgtccatc atcgcctcta gcactacttg gagacagatc 20460

gatgtcgctc tcttatacat cctcgaaacg ctcacgaatc tgaacgagtt tacttggaaa 20520

gcttcatcgg tctcatcgca cccagctcgc tgggcggagc taccccatct attgcaggac 20580

gcagtaaagg atgcaattcg gcgtccttcg ttgacctcgc tagttctcca tttcgctgac 20640

ttgactgaac tgttaaatac aacgatcatc gtgtcaccct ctttaaggca attaaggcta 20700

cagggcactg gctattcaaa tctcaccttg gaactgaccc gtcccagcat aataccttgc 20760

gacgatgcgg gtgaaccaaa tctgcaggaa ctttttgtca cgtcatgcaa gctagagtgg 20820

attgatgatg acgagatgtt ctttggctgg atgaacactg tccaaccgca tctcagcctc 20880

acgcgacttc acaagctcgc cgtgctccgc tgcatactta attcgacgca agtagagctg 20940

cccctggggc cgttgttata tcacgaatcc gttcggtcga agctggagga gttatggttc 21000
gatcttgggt ccacgcgtgg tcctccagct ggtgagctag tctcttcgat ccgtagctag 21060
gcgcattagg tcctcatgac gtttttggcc ttcaggtact gtatatctcg accagctgcc 21120
atccttacga cgacttgggc tgaccgcaga gctttcgggt atggctgcca cctggaactt 21180
gttatctgga attgctcagt ttttcgaggg cgagaacaac gttgagatta ctcaccttgc 21240
ccttgtcttc agcttctcca ctacttctct cgtactcaac ccgctcagca tgccgtggtc 21300
gcgattggac gcagctgtcg cagcgttgcc ctacttgaaa tgggttggga ttcaggtcat 21360
cgattgtaga tgccagcctt gcatgatgat ccctgaaaat tcggaatttt tgctgccaat 21420
gcttgcggag cagggagttt tgcactggtc aacgagcggg ttggagtttc cagcacaaaa 21480
gtagctgtca ggcacaccgg caattcgtac cggttttctt tcagccgctg acttggtttc 21540
aggatcgttt ttcacagaat tcaaatagta taccagcaaa acatggattt tgattcggca 21600
tctgtcacta tacaaccagt cttccttcta aggctctcac atcttgtccc atctctagac 21660
tctagtagta atgatctcca aattttcacc ttgaacgaca agttgtttta tttcaaagtc 21720
cgccacctga gctcgcgagg atttcaccct tgcggcagca ctaagcatca gtgattcctc 21780
aaatctgttt gctgttgcta tgaaactgtc tccgcctatt ttcatgttct attgatgaac 21840
aataatttaa aaaagatcat catgatttct agagtacaat ataaagtcag ggtgaataca 21900
gtgtatggta ccagcaccaa ccgctgttca ttgtcagaac catgagaaac cttgcgacat 21960
ggtaaagacc ccattccttt ttaccttgta tgtttgctaa ttaattttag gctgtgcctc 22020
tttctcctaa tgcctttatc ttgtttcgga actacatttt tgaattcagt tccaaacatt 22080
cctgctggtc aagatacatt atttaacaga tcttatattg gtttagacac agaagatatt 22140
ccgaatgatc ttcccttgac aatgtgcaac atctcaaact gcattggttt tggtggctat 22200
cagtcatcag gcagtagttt ggatttctgg gtgttcttca gtgaaatgga gacaaacctt 22260
aaacttgcat gggggtatgt gatatttgaa cttgacaatg gaccattctc accaaattgt 22320
aatcaagatg gtactttgta ttttctgaac gctgtctgcc atcagtacaa tgtgcacaaa 22380
tgtaatgatg ccattgtcat tgtctcacta tcaagtgtgg tcaactgaac ttgttcagat 22440
ttagcaattg gttcttttat gggacaccag aggcagattc acccaagtaa ggcttaattc 22500
atatcataga atttccagac ttgatgctca cacacagctc accatataac acaaccctca 22560
gcacaccttt gctccatctt gaagcaatat caaatactct ggctgaagat ttctatttga 22620
attgggatat tcaagcagtg ccagacaaca taccaattta tcttgcaatg agcagcatag 22680
aaacttcctt tgaatatcag gcggatatat caactgcaat gaatggtact tggatatttt 22740
cctctgggga cttctggaaa cccaatagta agccatatga tgggtgagtc atgagtcctt 22800
gttcattatt tttgaatgac attgatttaa ttcaaggtgg tatcagatat cagtatactg 22860

gtgataacca ctgttacttt ggatattgag gttatgtctg acattcttta tcatactagt 22920

aattatgaga ataacagtga taccattgca cctattattc aatcagcaag taattggttc 22980

tattatggta ctgaatttgc aaagtaggta cacaatctac atgcattatt ctgctagttg 23040

atcaaaagat atagtccaac aacatctagc ccacaaaatc agacaatcaa tgctctatct 23100

ccaactgctt ccaatggcac ctctattgct gccaagaaaa catcaatagg gccaatagtt 23160

ggaggagttg caggtggtat aattcttact atggttgccc tatttgtggt ttatagaaga 23220

tacacaatga gacgcaagtt gaggacaacc atgggttttg ggcaggaacc actcagcatg 23280

gtacaaattg agccttttca acttccacca tctcaagttg aactccaacc aagcaagttg 23340

caaaatatat ctatgcaaca ggtatgttta tttgtctggt cttttctcac tacagtctta 23400

attgctatca ggatgttgca agggccattc aacagacaag tgctgttcca gcaggaaatg 23460

atgctgaaat ggaagaaata ccacaggaaa gggaagaaga gcaggagttg ttgcgtgagc 23520

tgcagatttt gcggcgacta caggagatac ggggtataaa tgatggtcca ccaacttacc 23580

actctgatgt atctgtgtag atcattgcat aggtactatt accagtctga cagatctgtg 23640

tagatcattg caatacgtac tgttcttgaa atactggtaa aaagtatatt atatacagaa 23700

tttctggaca atgagatcct atatgagttg taaaatagca ctgtatctac taacatgacc 23760

caattttcat ttccaacgtt cactttactt ctcttttcta ccaggaaagg atcagagcaa 23820

agcctagcac accgacaata agaaggtgaa tcactattac tgtatacaat cagcatatgt 23880

gagcaaagga agattttgaa tgaggactac aggttttttg taggtttttt gtttactatt 23940

tctataagaa aagtccgata atctagacag gtgatgactg agtaaagaac gtggtgagga 24000

aaaaagaagg gatagatgaa gagaaagatg tatcgtacag atatgtcata tatataagta 24060

cctccaaaga gaacagcgtc cccacctgaa tctaacctcc cctgccgttt ggtatcatat 24120

tggtatggag aatatacgat gataacgaag agcggtcata cgtagaacgt caaacccgcg 24180

aaatgtacag ggtggacagc aatagtaaga gtgcagtaaa gtgaaatgaa atgagagggg 24240

ggagaggggg agagggaggg ggtggggggc aaggcaaaat aaagtatgaa agcaacgtaa 24300

gttcagcaaa gcattcgaat ctcaatggag aagaataagc acggtacaga caatccctga 24360

atatgaatga aaatgatgag gaggcgcaac gccatgtgtg tagtgttcgg ttgagttcag 24420

ttcgaaatcg gtaatctcga aggattagac aatgaacacc tcgggtatga acgaattaag 24480

gaataagaat gcggacggag agaaattata gagcatggag ttgaaaagga aagggcaagc 24540

gtgttaacat ccatcatact aataacacaa aaaaaacatc agctcaaact cacgcacaaa 24600

tcacccgaca acaaaacaca cctgagagtt cttaggttta tcatacatcc aagcagtcgc 24660

gttagggtct gggaacgaga cgtgtcagta acgacaacgc cgaaggtgcg taaaccaaaa 24720

ctcacttcca tacgcctcct tatgactctg tatgtaatcc gccgtaggat accccgtggg 24780

attacaccca acgttcgtct gtcccttccg ctgataaact ctcacataat ccacgagcat 24840

ctcagcgggg aaaatcatcg atgtcaggtc aatggtttgc caattttcta gtcggagcgg 24900

gataagattt gcagagagga gaggaagcgc gaacaggaga aagcaacaga attatcatca 24960

gtacctgaat gcacgaaacg ctatttcaaa gaacacatac gactaattcc caaattcaga 25020

acgatactca tcggctcctc cggtatcaac ctttgtccta caccacttcc attggttcct 25080

tgatcagggc caacagaagc agcacccata cgagcggtag gctgaccgtc gacttgccag 25140

gttatgaatc cgtccgagcg ggagttgggg tcggcccagt attcgaagcc tgttgcgtgc 25200

gcatagaacg acgacgaaag caacgaggag ggaatagaga gagaggagca agcagaggaa 25260

gagaaaatca gaatccattc aacgcatatg agactagaag cgacgtacca tacgtaacga 25320

acttcttgcc gctcccttgg aacccatcgt ctggcaccga gctcaacgca gaaacagctt 25380

gctgaaccgc acttccgtga tacgaattaa tcctcgtctt gttcgtgtcc catatcttga 25440

attcatcttc cgtatcctag tatcgtagaa acatgagttg attgagctta cttcgacttg 25500

taaaaaaaaa ggaacaagag gaacatcggg ccgtcgaaaa aagaaaatac gacacgctcg 25560

aacagtgaaa aggtcagaaa gcactcacgt tcaagaagac gtaatcatgc gagaatggcg 25620

caaactgcgc agactgcgac acgacctgac ccacattcgt cactttgtcc ttctccgctt 25680

cgaggacatc aatctccggt gcgcctcttc caagccacgt acccgtgttg gaccggaacg 25740

gccctgggtg gtcgggcgtg tacgatgatg tggcagggca tgtgcatgcg ctggacgggt 25800

ggagatgcag gtgtgtgaag caggacaagt ggaaagagga taatggtata atgggaggag 25860

gtacaggggg caaatgagaa gataaagggg aagaagagat atgaattagt acaagaacac 25920

gaattaaatt ggattataaa acacttacga caacctctga cctggtaacc aactcaactc 25980

gtagttgtac tttgacctcg attggtcaga gtgtaaagcc gctgcgggac ctgtaccgtc 26040

tttgagcgtt tgattgggga aggtaccgac gtcgcatgag tcatagctga gaagaaaggg 26100

acgtcagtga tatcttcaga gaagatgata tgaagcgccg caagaaagaa aagaaaaggg 26160

aaaaggagga aaaaatcaat gaatgagaaa ggcacgatcc cgtccaccac tctttctacc 26220

gcctcctttc ttcccactct tctactcctc ataactccct ccacctccca ttctattaat 26280

attccatttc gctagcgtca cagtcccgca gcaattcagc accaccaaaa ataaccgcga 26340

aaactccacc cagagtctcc atctataaat atccactata tctttgcgat gtatcgcact 26400

gctctctccc tttcccttct ctcctccacg accacgagaa ttccgcctgc agcctcgttc 26460

aacgtacacg gtgcaactgc cgccactcat agcatccagc ctgggtccag cgcaacatac 26520

tcaactctcc ccgcgaatcc caaccacaaa aacaaaaccg tgagagaagc aaaagacaac 26580

cccgacagcc aaatccacct cgaagaccat gtcaaggccc cacccatgct caacattgcg 26640

agaaagccag cgtctggact acagcctcca tttcgccact gaagcacgcc acttcgcatg 26700

acgttatcct cgtaggacga ggacgtaacg gacatggcaa aggaaacctt gaggtctcga 26760

atgtacgcag gaaaagcacg agaaaccgta gtcattccag aggccacgtc acctgagcac 26820

caccaacata taacgccgag caaaagaaaa aaaaaacacc acgaaaagat tcaaaacaag 26880

aaaatccctg aactgaactc acgtataagg ccacatccca tccgtcgtcg caccataacc 26940

aggtctaccc agattcccca tcgtccacgc accaggccaa tacccctgcg tgttggcatc 27000

tggaccagga agaacgatgg atacctcaat atatccacct gtgaagaagg acgtgagtga 27060

gggtagaaga gacgagaaat gtagtagagg tatggaagag ggacagtatt ggccaaaaag 27120

aaaaccttca actcactcga aaaacaaaac ttattccagc tctgcaacat cccactccta 27180

tacgtcaaat tatgattact cccaacacca aacggcgccg tcgatccttg cgttaaccca 27240

ctctgaaccg tattcgctga atccatcgtg atcactaggt acccatcacg agtcgtcacc 27300

tgcgatgggt cgtaccattc taaatcggca gtggaaccgt accataggtc gacagcttcc 27360

cagtaggcgt cgtcgccggg gtagaatgtt cgtcccgggg tttcgaactc gtcggagaag 27420

acaaggtcgt aggtctcgcc gtcgaagcct gtgcgggtgt gtacagaggt aggggtggcg 27480

agatcaatga gggtcgggga gaaagggaag acgggtagag gggcttggcc tgttgagttt 27540

atcctggaag agggtgttag tgggaggaaa aagagaggaa caggaaaaga agaggcgtac 27600

tggatgtttc cgtctatggc ttcgttgaac tgctggtcat ggtagaacct tacgaccggg 27660

taaccgacaa agagagcgat taagctagtc attgagaacg ttgtgagggt actgaagaag 27720

aaaaggaaga aaatggcata cgccaatatt ataacaacca acaagctgac attcgctata 27780

cccctccaag gaaacctccc accacctgcc aaccctcctc tcgagaacac ctgagtcaat 27840

cccatgagta cgcgaaccca cacacgcata cgacccaacg aaaactcacc ttcttaccag 27900

ccttttcatc aatcgtagga tcatgcaaca aatccatctc atcgacttcc ccaccagcat 27960

ctccatctcc atcctcaaga ggatcatacg catagggtat caaagtcttc ggtctccccc 28020

cacctgtcca cttgtcatct ttttccccat cccctgaccc ggccacaggt gtcggatgtg 28080

agataccgcc agttccactg cccaaattca ctgctaggta gccactaccg tgcccaccgt 28140

atattgatgg atatttcgac tcgtcagcgg atagcgatac aatcgaggag cggttcgacg 28200

agctagcact gagcgtcgat tcgcgacgga aatgcggtgt gggaggaagt agagcatggt 28260

ctgcacctgc tgagggtaat ggtggtggtg aaggtggtga aggcaaagaa gtgttgactt 28320

gaggtgaagc tgctgcaagg aggttcgaga acgtggaatg tcgcgataga gatgtaggag 28380

aagatgacgg ggacacgaaa tctgattcat cagcaaaacg atagagaatc atcgagcctc 28440

tggacggcga aaacccttgt ggttggtcgt tgtatgcgcc tggaattggt ctcgtactca 28500

tacgtggggt acccatgaga ggcatcgaac tgactcttga tgcaggtcga gatgtcagtc 28560
cgactgttcc tactcctgct cgtgagccca tactcgacaa agtcgaacct gagttagacc 28620
cggaacgagt cgacgaagat tttcttccct ccgagtctac atcaggctct tgtgagcctg 28680
gtgtagcaga gctttggccc aatggactcc gtgtaaagga agaggtcgga cgtggttgcg 28740
aagggtgtag tactgcagct ggtgatggtg aaggtgaagg tgaaggtgag gaagatagca 28800
gttgctgtga ttgtccctcc gtcaatttct ccatgttgta aaggatagtc ctaaagaaat 28860
gtcaaaaaga agaacgaatt tcgtgattta ttactcactg gaagcacgaa attcaacgaa 28920
cgaggtcacg aacgaaagaa agtcgctcag ggcctgatgc agtccctctc ttaatccaat 28980
tcctaatttt ccccagctac ctgatatata tcaaggaagc gaagagcaga gcgacaaggc 29040
ggacagtgta tcgtatcacc gtcgtagcct atcgtatctt gctgattcgc ctcagagcta 29100
ggattccggt cagcgcccgt cgaagtcgtc cacccacgtc gtccgtcgtc gtcgttgtcg 29160
tgcggaaaaa cgcagcaaaa agcaaccgag cctcacccaa tcaacacact tccctcttct 29220
tcgagctcgt gactgaaatt aggaagggaa aagagaagaa aaagcacaca agtcttggga 29280
aatgaattca caaaccgggc atactaaacc gtgtaacaaa ccgactttca ccggccggca 29340
tcaagggtac acggcagtca ccggcccggg gagccaagag gaaagaaatt agtttacata 29400
atgaaaattt taaatttcat ttcaactttc actacataca ctatcaataa tcagcaagtt 29460
tcaagggaag tgcaggctac caaagaatac ttcactgctg ctgaacgact cagattggcc 29520
actttacagt caagatagag gatgacgaag gaaccggcac tcagtgtaca gcgatattcg 29580
gcgcggctgt tcaggcatcc aactttcaat ccctcaacgt caacgtcaac gtcaacgtcc 29640
acatcaatta tcgggaaaga gctttgaaat gatatctctc aagttgaatt gcacaagaac 29700
ttgcgtactt gccggaggtt ggcgttgttg tcgagctcat tcttgagtga actggacgac 29760
gttgtatctt gtaagtcatc tttgcatatc tacactacct attaacatat ctaaacgctc 29820
cagatactta tcctgattta tgaacgcaca tatgcgagtt cagcttcagt actcagtgct 29880
cagccctcag ccctcagccc tcgcaactgt cctggtcaaa gctaaacgcg gacaaacttc 29940
cggaatcatg ggtacaacgt agctcccggt ggactcgaca ctgctatcca cggttgtttc 30000
caatgtaagt tcgcccagaa cctgcaccca cactactggt tactcaacac cacttcaggg 30060
caggcttcgc aaacctaata catctcagca aaccttattc aatcgctctg atttaagtag 30120
tgtggcgcgc gctattctcc cttcttttct tccacacccc atgtcaaatt ccaaccaaaa 30180
aagtcccaca agcagctcga aagaatcctc cacgccttgc agattcttcc tcaaaggaca 30240
ctgtaaattc ggagagagct gcaatcgaag tcacgatgtg aaacctaggc ctgctgtcca 30300
acgtcgaagt tggatgcaat cattggctag caatgggggg cgtttttcgg tgagctatcg 30360
tgtatctatc aagaaagatt ataactgaag tgctctcttt tattgctggc cccattagat 30420

caattcccca tcgacatcgc aacaaaggca cgacgttact ccggagcctt ctgcaggtat 30480

tactttcggc aacttgactt tcacgaatga agacctttca gaggcaagtt atcgtcaaac 30540

tactcaagta attctcctct catcaaccca ttagaacgac tcctcatcgg catcggcact 30600

accatcggag aaccacgtgc acgtctcacc accgtctatt gcggtgaaca cctcaacaca 30660

acccacacgc ccccagaacc aaaaacaaaa caaaccttgt ttcgcatgga gagccgggac 30720

gtgtcgtaga ggggatacct gtaaattcaa acacgatcca gaggtgaggg gccgcaaccc 30780

ttccagcgcc tttttttcga gtcattctgg cttctggcgc taatatacga tcacatcatc 30840

ctcatctgaa ttaggatcag gtgcagcagc agcgtgtatc ctcacctgtc actgattcac 30900

ctgttctcgc ggcacacgag aatggcgaaa cacgcggaca gaccggcgct cgacgtcaac 30960

gtgcggataa ttggagggct cagcgtggag cgaaaggaac aaagaatgct caggatccaa 31020

ataattctac gggtactgct aatcaacagg gctccgcggg aggaattagg ggcccacagg 31080

attccccgag aagcgggggt ggcggtggat ctgttcctcg ggaagaacag ctccgaattc 31140

agagagaagc tcgacgtgct gtgcaagaag cagaggagga ggaaaggcaa cgtaaaactg 31200

ccgaagaaat tgctgcagaa ctcgatcgac aagtggagat gtggagacaa cgtagagcag 31260

ctgaggcgga acagctacgt attcagcgcg aagctgagca tgctgagagg gaagcacaga 31320

gaaggaaatc ggaggaggaa cgtgagcagc ttcgtattca gcaggaagct cgtcgagctg 31380

agcgtgctcg aagggaagaa gcattcacta tccgacaagt cgagcttggc agcaccatcg 31440

tcacgttcag tgccggcgta gctatcgatc acatcgttac tgggttcgag tcttgtcgcg 31500

tgcaagtgaa gaatctccca tttgatgcga aacgtgatga agtctacgaa atgttccgag 31560

cgcagggtgt tgaggagggg aaatttcatc ttgtgaatat gaaaagggtg aaccagcgac 31620

aggaggcgag tttcattgtc gatggggaag ttgggaaggc aatcgctctt gggcttgatg 31680

ggacagattt ccgtggtaat gccctcgatt tcagtgtcag cgacagcgga actatcggcg 31740

ggatgtattc tacgggggac agtgctgtcc tgaccgttca ttggagagct cctagcgcgc 31800

gcttcgtagc gagctatgca tatgctgaaa gtgccgatgc gaagatgcga gaggtgaatg 31860

agaaggtgat caatggtcga aggatcagag ttgagcgaaa tcggacgcgt caagggtcac 31920

tcgatccatg tgcactctta atcagtggtt tcacaccgca aacatcggac gaggctgtga 31980

gggaagcctt tcaggctctt tctgtacgaa ggctccccca tgctattgaa tacgatgaga 32040

attacgcgaa aacctacgtc aggaatctca ttaccgtaat ctcaaatgta cggtttgaag 32100

ggccaacact caattctttg gaaggcaatt acaccttacg tgctcacttc gatacttggg 32160

accaaaccaa gttggctcgc gatcagtttc atcaggggag gtatccccag atcggaaatg 32220

gtacctttcg actacggctt cccgatccca tccagtacaa aattaccatt ccttctcaac 32280

agcataaagc acagaagaaa caatgggaca ctttcgtctc agatactgcg ggccaaaaag 32340
gaagctatgt tctcatcacc cagcaaaacg attccgtccg gacttttgtt cgcgtagggg 32400
gagatgacag tcaagcagtc ggtatgctca aggttcgtgt cgagcgcttg gctgcgggcg 32460
aaaagctaga tttatggcat cgttcgtttg cgaatgcagc cggacagaga tttctcgagt 32520
ctatataccc tgctgtggga gctctcgtca tgcatgactg gagaatgaag attgtcaaag 32580
tatacgggga accttcagcg attgcgaagg ctcgagagat gattatggag gagatagaaa 32640
ggctttctgg acttgaatat actgttgcgc tgaagagaca gtctatccgg tttttcactc 32700
agcgtggaat cgcttcgctc aaagaaacct ttggagaaga tagcgttacg ctcgacatct 32760
ctacctctcc ttgtcgtatc accattagag gcggggaaga ggctcgtcat gctctcagcc 32820
gtctcataga tgaatctcta gacgaacttg cggttgctaa tgctgcaccc aaggacgaaa 32880
gctccacatg ccctatctgc ttagacgaag tctcaacgcc agtacaactt ggatgcggac 32940
atgaatactg cactgcttgt atacgtcact tcttcactgc tgacattaag acgttcccac 33000
ttgtctgcat ggggaacgag gcaacatgct tgaaacccat cgctttaccc accattcaac 33060
gattcctcac accacagcag ttcaacgttc tcctcgaaac cgcctttgtg agtcacatcg 33120
agacgaaccc gcaaagttac aagtactgtc cgacacctga ttgcgtgcaa atttatcgcc 33180
gcaccacaaa cgataccgct gctcttcagc tgcgttgccc gtcctgtctc attacgatct 33240
gctccagatg ccaagaaggc catgatggga tgacttgtga agagaggaga atccagggcg 33300
atccagcaga acaggaacgt ctgaatgaag agtgggcgcg aacggcaggg gtgaagaaat 33360
gtccttcttg caacgtctgg attgaaaaga ccgagggatg caatcacatg tcgtgcaaat 33420
gtggtgctca tgtgtgttgg gtttgtttgg gtatattcga ggcagggact atctatgaac 33480
atatgggaag ggctcatgga gggatctttg aagacaataa tcgcccgagg gtagtgcagc 33540
agcctgaacg ggtgattgac cgagatgacg atttcgattt tctcgaacaa gcacgccggc 33600
tacgggttct cgaggctaat aggttgcgcc gacaggagga tgtgcgagtg cagagggaat 33660
atatcctagc ggatgaagta cgaaggagga ctcaagctca ggctcaagga atccgagcat 33720
tgagagaggc tgaacggatg atagaggatg gggaacagag aaggagggct caagagtggc 33780
aacgacaaca gcaagcggcg ttcgcccaag agcgtagatt gcaactgctg agagagcagg 33840
aacagcgcga gcgtgaaagt aggcgagatg gaggtggttg gggttgtact gttatgtgag 33900
atgtgtttgt atgtaagata tccgatgatc gatgatccgt aatggtcatt tgttttgaac 33960
gtgcatcact cagagactta tcatcgactc tcctgatgaa gctaagtgta ttaaacatac 34020
aagaactctc actcgtacac caagcggctt gagaccgaaa tcagtcgagc tatcaaggaa 34080
tcagtgtatc cgagagatca gtccttaata aaccttggtc tcttccgtgt acccgccaac 34140
tacactcacg aacggcgaac cgtgagtgaa aatgtaattg attttgttgg acaagtacct 34200

ggtgcgacat aaaacatcgt ataaggtccc gtgtagtata gatctcgacc ttctaggcag 34260

ctttgttcct ggccattggc ccatgcagta ttcgtgaacc cggttgactg acataacccc 34320

aaggtatcat tcacggtggg attcaatgcc tttagtggta ctatggtaga atatcagtga 34380

gcttttcagc agaagaggta tcgaaattga ttctttgaag gttgactctg aagcatgcac 34440

gcatgccaca taagtcattt ctgctcttga tgttccaagc acacatacct acctacggtc 34500

cgagtccact ttaacatgta ctgtttcttg tgataatgtc cttgacaaat ggatctatgc 34560

aggtatacaa ttccttccct cccccgtcaa gatggcatcg tgtgtctccg cgatcgccgt 34620

tatatatgta ggtcggcata cagcggaagt tcgaaaagag tacgcatata aaggtgtatt 34680

tcaagtacga agctatccag gcaaactcca gccacttctt cctaaccttc gtataaaggt 34740

gaaaagcctg ttctctgtct cgaactgaaa actcactcgt tatcattcat ccagtttgat 34800

tatcatgatg ttcggtggtc gcaacctcac tcttcttgtc gctgctgtgt tcgcggtctt 34860

gatccaagga ggtaagctaa atcctgcaca agttctcgat tagatgccaa catctgatat 34920

cgtcgtgaat gtagctgttg ctagccctgt aggacctgcg gaagctgcgc tcgcagcccg 34980

caatgaggaa tgcggtgcag tttacgaggg cgagctctgc gtaagctgct gtggaggtcc 35040

ctaggtctat cacaaataag tactgtcatg agttatccaa acgtattctg attcatcacg 35100

tacatggaaa aagcatttca atgaactcgg tggaggcatt cctgcttctt ggctactaga 35160

agaatgcgct aagatgagct acgactgaat gattgagccg tatacacaac ttcaacagag 35220

gttttccgag tcgacgcgag gctgtccctg aagtggccct actccctctc catagctaac 35280

gtttcgcgtg ttattgcact cgcacttgta gttacgagtg cagggaacgg agtgaccgg 35339

<210> 14
<211> 43
<212> DNA
<213> Omphalotus olearius

<220>
<221> ophMA_fw
<222> (1)..(43)
<223> primer

<400> 14
cgtcgacgag aacttgtatt tccaggagac ttccactcag acc 43

<210> 15
<211> 33
<212> DNA
<213> Omphalotus olearius

<220>
<221> ophMA_rev

<222>  (1)..(33)
<223>  primer

<400>  15
gcggccgcat tattccgtgc tcatgactga tcc                                    33


<210>  16
<211>  42
<212>  DNA
<213>  Omphalotus olearius


<220>
<221>  ophP_fw
<222>  (1)..(42)
<223>  primer

<400>  16
ctaattattc gaaggatcct acatgagcga taaaattatt ca                          42


<210>  17
<211>  40
<212>  DNA
<213>  Omphalotus olearius


<220>
<221>  SalI_rev
<222>  (1)..(40)
<223>  primer

<400>  17
agaacgaaaa tatcgttatc ctgccgtcgt cctgatacca                             40


<210>  18
<211>  40
<212>  DNA
<213>  Omphalotus olearius


<220>
<221>  SalI_fw
<222>  (1)..(40)
<223>  primer

<400>  18
tggtatcagg acgacggcag gataacgata ttttcgttct                             40


<210>  19
<211>  41
<212>  DNA
<213>  Omphalotus olearius


<220>
<221>  ophP_rev
<222>  (1)..(41)
<223>  primer

<400>  19
ggccgcccta gggaattcta ctcaaactgt tacgcaggac c                41

<210>  20
<211>  1367
<212>  DNA
<213>  Lentinula edodes

<220>
<221>  methylase
<222>  (1)..(1367)
<223>  chromosomal DNA

<400>  20
atggagactc ctaccttaaa caaatccggc tccctcacta tcgttggcac tggcatcgag      60

agtattggtc aaatgaccct tcagaccttg tcttacatcg aagccgccga caaagttttt     120

tactgtgtca tagaccctgc caccgaagca ttcatcctca ccaagaacaa ggactgtgtc     180

gacctgtacc agtactacga taatggcaag tccaggatgg atacctacac ccaaatgtct     240

gaggtgagat gtttcttgtc aaatgttttc tttgtgttct gtatagagct gacatcttca     300

aaggttatgc tcagggaggt ccgcaagggc cttgatgttg taggcgtctt ctacggacac     360

ccaggtgtgt tcgttaaccc ttcgcttcga gcgctcgcta tcgccaaaag tgaggggttc     420

aaggctagga tgctgccggg cgtatctgcg gaggactgcc tttatgcaga cttgtgcatc     480

gatccttcga accccggatg tctgacctac gaagcatctg acttcctcat tcgagagagg     540

ccaacgaaca tttacagcca ttttatcctt ttccaagttg gatgtgtcgg tattgctgac     600

ttcaacttca ctggatttga agtaagtatc atcaatctcc atgaggtata catttaataa     660

actctttgtt ctagaactcg aaattcggaa ttcttgttga ccgcctcgag aaggagtatg     720

gcgcagaaca tcctgtcgtg cactacatcg ccgctatgct ccctcacgag gatcccgtta     780

ccgatcagtg gaccattgga caactccgtg agccggagtt ctacaaacgc gtcggcggtg     840

tttccacttt ctacatcccg ccaaaggagc gaaaggagat taacgttgat atcattcgtg     900

aactcaagtt tctccctgag gggaaagttc cggatacgcg tactcagatt tacccaccca     960

accaatggga acccgaggta ccgactgttc ctgcgtatgg atcaaatgag cacgccgcta    1020

tcgctcagtt ggacactcac actcctcccg agcaatatca acctctcgct acttccaaag    1080

ccatgactga tgtgatgacc aagttggcgt tggatcccaa ggcgctcgca gaatacaagg    1140

ctgaccaccg agccttcgct cagtccgttc cggatttgac ggcgaatgag aggaccgctt    1200

tggagattgg agactcctgg gcgttccgct gcgctatgaa ggaaatgcct atttcactcc    1260

tagacaacgc caagcagtca atggaagaag cctccgaaca aggcttcccg tggatcatcg    1320

tcgttggtgt cgttggtgtc gttggttctg ttgttagtag cgcctga                 1367

<210> 21
<211> 3296
<212> DNA
<213> Lentinula edodes

<220>
<221> oligopeptidase
<222> (1)..(3296)
<223> chromosomal DNA

<400> 21
atgttttccg caacccagga gagtcccact atgtcagttc cgcagtggga tccataccca 60

ccagtcagta gagacgaaac ctcagcaatc acgtatcaga gcaagttgtg cggttctgtt 120

actgtacgcg atccgtacag tgcacttgag gtgccttttg atgatagtga agagaccaaa 180

gtttgtggaa atcatgattc cgtgtacagg atcctcaatg atcaggatat aggcctttgt 240

tcatgcacaa cgcaagtttg cgcggacgta tctagacgag attcctgaca ggtatatgtc 300

taatgtgctc tttatcagtc actaacgctt ttatttgaca gagaaacgtg gctgcaaacg 360

ctgaaagaga gttggaacta cagacgattc actgtcccca aacgcgaaag cgatggttac 420

acctacttcg aatacaacga cggtcttcaa tctcaaatgt ctctacgtcg cgtgaaagtg 480

agcgaagagg acaccattct caccgagtct ggacctggtg gggaactctt ctttgatcct 540

aacctgctct ctctggatgg aaacgcggcc ctaactggct caatgatgag tccatgtggc 600

aaatactggg cttacggcgt ttctgaacat gttcgtgtgt tgtcataagg gagcactttc 660

aagttttact gatacaacat gagaaagggt tctgattgga tgacaacata tgttcggaaa 720

acatcgtcac cgcatatgcc ttctcaagag aagggcaaag atcctggaag aatggacgac 780

gttatccgtt actctcgctt ctttattgta tattggtcga gtgacagcaa aggtttggtc 840

gcaatgtgca aatggttatc aaagaggcct acttattctt aacggtctag gtttttttta 900

ttctagatat ccaccagaag atgacgaggg caaaggcaat acacctgcgc agaattgcat 960

ggtgagtttc tttccacgct ctatgtttac tactctcatt tccaacgcca tcaggtgtat 1020

tatcaccgcc tcggggagaa acaggaaaaa gatactcttg tctagtaagt tcatgaggca 1080

tcaacacatg gccttgctta tgaatattct gaaagtgagg atcccgagca tccattctgg 1140

ctctgggcac tgcagctctc ccctaggtta gtgtttccca gcactcctga tcactgtatt 1200

cacttagcaa atatgtagtg gccgatatgc actcttgact gcaagcgtac gcaaaactca 1260

tactatatac acatctcact ctaatgttta gctacacata gcgggatgct agtcatactc 1320

aactagccaa aattgctgat attgggacga gcgacattca aaacggtatt caatggttga 1380

ctatccatga tcaatggcag gctagatttg ttatgtacgt ctaccttctc ttctgcttct 1440

cgacctcgac taacattcta gttaatactg atctcattag aattggagac gatgattcaa 1500

caatttattt catgacgaac ctagaagcta aaaattacct agtagcaacc ttggacattc 1560

ggcattcaga agcgggggtc aagacactgg tggctgaaaa tcctgacgcc cttctcatat 1620

cggcaagcat cctctcgacc gataaactcg tcctcgtcta ccttcataat gccaggcatg 1680

aaatccatgt acacgatctt aacaccggaa aaccgatccg ccagatattc gacaatttga 1740

tcgggcagtt ttcgctgtcg ggacggcgtg acgataacga tatgtttgtc tttcacagtg 1800

gtttcacttc tcccggcacc atatatcggt gagctcgttg ataagacatt gtggcaccct 1860

cgccgacatt gtacaggttc cgattgaatg aagatagtaa caagggcacc ctatttcgtg 1920

ctgtacaggt tcctggcctc aacctgagtg atttcacgac agaatcggta tgcatacgtg 1980

cttctcttca tcgctcctat tgctaagcag gatatgcagg tgttttatcc atcaaaggat 2040

gggactccgt aatcattctt gctgaatctc ttaaacatcc gaagcctgat aataaattta 2100

gtattcacat gttcattact cgattgaagg atacccccgt cgacggaact gcccctgtat 2160

atatttatgg ctacggcggc ttcgccctgg caatgctccc gaccttctct gtctcgacgc 2220

ttctgttctg caagatctac cgggcaatgt atgtggtacc caacatacgg taattaattc 2280

atcatcgttt ccctttcggt ctctgaattg gcttatgctt cagtggtggt tcagagtttg 2340

gagaatcatg gcaccgggag gtgagtagtg tttaattttt gcgtaaaaaa taactttgtc 2400

tcaggcgcgt tctacaggga atgttggaca agaaacagaa cgtgttcgac gacttcaatg 2460

cagctacgaa atggctcgtt gcgaacaaat atgccaataa atataacgtt gccattcgcg 2520

gaggatccaa cggaggtgag ctgagctgta actgttcgaa cctttgatca cagacattaa 2580

gtgatgtggt ttttctacag gagttttgac aactgcgtgt gcgaatcaag cgcctgagct 2640

ctaccgctgt gtcatcacca ttggaggtat catcgacatg ctcagagttt gtcaccatat 2700

ccttcgttct gattcctcct aattcctcac tcatccatga cattgcagtt tcccaagttt 2760

actttcggcg ctttatggcg ttcggaatac ggtgatgtac taaaatgtca attggtctga 2820

aaaaatctga ccctttatag cccgaggatc cggaggattt tgacttcatt tacaagtgag 2880

ctgaacagtc ccgatacaag attgcttcct tacccaaatc ttgacaggta ttccccttat 2940

cataacattc catcaggaga tgtagtcttg ccagctatgc ttttctttac tgctgcgtat 3000

gatgaccgag tttctcctct gcactgtgag tgggccaaaa tcttgaggtc ttatagaact 3060

ctgatcggtt taaagcattc aaacatgttg ctgccttaca gtataacttc cccaacggtc 3120

caaacccggt tttgatgcgt attgatctaa acactggaca tttcgccggc aaggtttgaa 3180

attttttact gtggtctcgc actttgctaa ttccatccca gagtacacag aaaatgctcg 3240

aagaaaccgc cgacgagtac aggtgcgact tactctgttg taacctacaa ttgtga 3296

<210> 22
<211> 25900
<212> DNA

<213>  Lentinula edodes

<220>
<221>  cluster biosynthesis
<222>  (1)..(25900)
<223>  chromosomal DNA

<400>  22
ataattaatc ataataaggc ttgagttatg aatacatgag caataaatgt acgtacagcg      60

agatgcaagg agaaacgaag tttcgaaata tacattcatg tgtttcaact aagccaagtt     120

atatgtattg tatgcacagc agatcgaaag caagaaaaga atcatctacc gctggagaag     180

gagcccagtc tcagctagag atgcctgcat ctagtagcga caaccccgtg agctgactca     240

ctcacaatat gattaatcgt agatgacagg ctctcaggca gtagagagaa aagcataaag     300

gaaagaaacc agctacaact ggattaattc ttcatctata caaagtcccg tccacttcgt     360

tcccgtcatg atcaaaactg aacgacctta aaaacagatt gtgagaggcc cttggcgtgg     420

gtgcagcgaa agccgggata ggctcaggct cataaccgcg gccgtcatcg tagtcgccaa     480

cttcgttcaa gtctaagcct agggcgttga catctccggg aggaggacta tgaggagtct     540

gggagttaga gtcacgactt gcacggctag ggccgctgac agagccaccg ttaggcgcca     600

catgtgtcac aggtgccata ggaattgcag gtgccgagtt gtattctgga aaggctggac     660

gatggggagc actagccagt gcggaagaag gataaccgaa attaggtagt gtaggtaggg     720

ggttggaatt gagattggag gttgggatag ggcgcaaaga ctgaggacga tgaccacgag     780

catgatcata gttgcgggtg ttgtcgcgag gcgtcgtgtt gacagtgttg acaggaaggg     840

aggtgtatcg ctgatttact ggtctagtat tccccgtccc agcagattga ctgacttgac     900

tgacattacc acgaggagca gcgcgggagg cagtcggtgt ttcggagtca tgatgcgtga     960

ttgcgtggtt gttcgtttgt acgtcggatc tagcagaaga actaacgaca ggggctgaga    1020

aacccgccac ttcatttgtc ccaactgcag atggtcgatt cgggttgttt gcatttacag    1080

gaataacagg caggaatgct ctgggatcac ccaccggtat ctcctgagag gatgcaagac    1140

tcgcgcgact cgaatgatgc gcatgctcag gaaacccgcg cagattagcc atcacgtcac    1200

ttgcattgga ttgtcttcgg gatgaattag cggtcgttac tgtaccccaa gtgggcgtct    1260

cgctagggac agcggaagct cggttgttag caaaatgttg ggcttgggct tgggatgatg    1320

aacgaagggg gtcttccatc aagtcgcgga gccgattggc gaggctttct ggggtgatgc    1380

tttgtgttgt gggctgtgtt gtcgtcgtgc ctcgtctcaa tgcctgctgg tctccattgg    1440

aagtggttgc agaggcggta gcaggaggta ttgcggattc agtcgcgcca ttgaatctca    1500

ctgaccgagg aagtggagca gagactgggg ggccaggagt tgttcttgct ctcgcgctga    1560

atccactgtt caatccctcc gtgtacccgc tggcaaaagc cgcttgggga tcaactgcac    1620

gcctctcctc gaaggactca tctatcaacc ctaaaccagt gacaccagct atatcttctc 1680
ttccaccact cagatgctct tctctacgac gcgttctcga ttttgacttg tgtcgacgac 1740
ggtgtttgcg ggataagtcc tctactgggg cactggctgg tggaggaatg gaagatgagg 1800
catacccatt gacgtatgtc gtctgtgagc cggcttgaga ccagtgttgg ctggcaccgg 1860
gcggaacata gattctgtca ttttcggcag cgcctggaac gattctgtgt cttctagacg 1920
gcgaagagcg cgatgcatat tgttgcgcct gagaaacggc agcgtcaagt acgacgggaa 1980
gtgcttctag tcgagaagat cgacgcggac gagatgttag tggagggatg ggtagagggt 2040
aatttggaat gggtacgtag ttagcaggag gctccatgta atgttgagaa gacacaacga 2100
cttcgggagt gtgtggcata gcactggcaa ggccgggggc gacaagaccg ttggtgtctc 2160
gtggacggga agagcctgcg ccagctgatg aagttgcaag gtcggatgag gacccatccc 2220
agcctcgacg atcatgtata gatgcctcta ttgctgcagc gacctgttca gcttcgacag 2280
acgaactacg tatatcccct gcgactgtgg cgtatctttc catagtaata gccatagggg 2340
gggctggcag cggattgtga cgtagagaat cgttctctga agtgtaatcc gtgacccttg 2400
ctttgccttt ccctttgtct cgtctactaa attctagacg ggcaagttga ttgcgcagac 2460
tttggacttc tgattgggat tgttggagag ttctgtcatg atagtcagac caataagcat 2520
ggctgactcg gggaggactt acgtttcaag ttcctcgatg gtagtagaga aactggcctc 2580
cattgcgagt cccatatcac gatcgtgcac gttgctgcgt tgaagatgca ttaaagaagt 2640
ttccagtcgc ttgatcctga atctatcttc tgcttttcgt tctttgagtt tttggaagtc 2700
ggcttgtacc tttctaagct gtgtcaaagc aggactctga ggaaggtaca accataaact 2760
cagacgaatt gtggcaagtg acgagcacat catgcagacc tcttgatttt gtagccagcg 2820
ttctacttct tctaacgtat taacgacttc cacttcgtcg tcccaagcca taatcagttt 2880
ctgaacgatt aagctgtcat cactccctgg aagcgcatcc atgtgcaatt tcttgataga 2940
atctctgtta aatctttttc gacatagtgg gcatgtcgct gtagctgtcc tttcaagaca 3000
ttctttgcaa aaaatatgcc cgcatgctag ggagcaagtc acttgtataa taacacgaag 3060
tcttgtcaat caatgaaaac ttacgaatag catatggctc cttcagtgtg gtctcctctt 3120
gccaggagta tgggtccaag cacacatcgc agcagctcga cgggtggacg ataagcataa 3180
gagcattccg gagagtagtt gagacaaaaa gagggagctg cttgcgccac tcaaatcatg 3240
tacacgaggc aggtaagtac tattcgacaa gaaaggaaat tttgccaggc tgtgcagcct 3300
gtgctcacag ccacagcaaa agacttcatt acataattga ttataatttt caaatttgcg 3360
gaaattctac tctgctagtc gaccaatgca atattcccct ttcggtcaac attggcagca 3420
taatcttcgt tttgggagga ataatgccag tcggttcacg tctacaatca acgaactgga 3480
ctcccaccca gcgtaaggtg ggtattgccg ctacagcggc atcgagactc ccccatggca 3540

cctttagtgg gtctaataca agagaagtac tggaaaagct aaagcaaagg ataagatgag 3600
taattccagc tgcatttgta ttctcaaaaa atctagataa tcctaaaacc aaatcccaat 3660
tagcatcgaa gactcgtgag aggcccatag atagcccgag tcgctttaag gaaggcaagc 3720
ggtcgagcga catggtatct agaaatacga gatgtgaaac gactcgaacg tgaacaattc 3780
aagcgacaaa ccaactggag gaccattagt agagatcata tcgaaccaaa gttcttccag 3840
gtttgactga acagactggt ggtataataa cgccccccaa ggaagttcag gcaattttga 3900
ttgtagcgaa gatcggaaga ctgccagttt ctgaagccgt tcaagactga gattcggtag 3960
aatagcagtg atccacccaa agcacatctt ctcgtcctcg atccactcaa gcttacatgc 4020
cgtgacgaac aatctagacg gcatctctcc gaaaggtatt atactcggac gagtcagttc 4080
caaggcgatt ttggaaaatc ccgtgcctcg taagttcaga tatgtcaaag agggagagat 4140
aatgattctt gcattcaata gatcggtcag gtcggcgaaa tgaaggaata gggaagtgac 4200
tgaaggacgt ctgatcgtgg ctctgattga gtcctgcaga ggttggggta attctctcca 4260
ataagtcggg cgtgaggagt tggaagcggc tttccatatg aagcaggtta gatcggtgag 4320
agaattcaag atatcaacga gggcggggtc gagctgtgtc cagctaaaac cggagtcgag 4380
aatagataag gatcggatac tcgaaacgag atgaggtgac gatcgaagta gcttcagtgt 4440
tgcgtcactt gagcgttggt atcgtctatt tccttctgcc ttcgaacagg atatgatgat 4500
attcgagagg agatatcgtt ggcagattgg ggtaaaggaa gagcggacga gggacaagga 4560
tttcactgta gccttgtcct ttctggctag gagttcgcga attatagctt cagcgatgtc 4620
tagcggcagg acaagcattg tgtgcgattg ggcaagattc tagctctggg gaaatgagta 4680
tccttggtcc ggcgtatgcc ggatccgctt ctgcaagtga gcgtgctttg agtctggtca 4740
ctggtgttct ggtagcggag tactaaattc aaaaataaag aatttggagc atgtgtcgaa 4800
gttatgtttt tactcgggct gtactaactt tttgtggaat ttgtctgttt ctatcaattt 4860
tttgtcaggc agaatggaaa catcggaaaa gttgatggaa ccatcgtgtt agtgagttga 4920
cttccctccc tcaaaacata tcacaataag tatgtactta gcagcctcgc gattccttgg 4980
atagggcact aatctgttaa aggagagtca gtatctttca catgttttcc gcaacccagg 5040
agagtcccac tatgtcagtt ccgcagtggg atccataccc accagtcagt agagacgaaa 5100
cctcagcaat cacgtatcag agcaagttgt gcggttctgt tactgtacgc gatccgtaca 5160
gtgcacttga ggtgcctttt gatgatagtg aagagaccaa agtttgtgga aatcatgatt 5220
ccgtgtacag gatcctcaat gatcaggata taggcctttg ttcatgcaca acgcaagttt 5280
gcgcggacgt atctagacga gattcctgac aggtatatgt ctaatgtgct ctttatcagt 5340
cactaacgct tttatttgac agagaaacgt ggctgcaaac gctgaaagag agttggaact 5400

acagacgatt cactgtcccc aaacgcgaaa gcgatggtta cacctacttc gaatacaacg 5460

acggtcttca atctcaaatg tctctacgtc gcgtgaaagt gagcgaagag gacaccattc 5520

tcaccgagtc tggacctggt ggggaactct tctttgatcc taacctgctc tctctggatg 5580

gaaacgcggc cctaactggc tcaatgatga gtccatgtgg caaatactgg gcttacggcg 5640

tttctgaaca tgttcgtgtg ttgtcataag ggagcacttt caagttttac tgatacaaca 5700

tgagaaaggg ttctgattgg atgacaacat atgttcggaa aacatcgtca ccgcatatgc 5760

cttctcaaga gaagggcaaa gatcctggaa gaatggacga cgttatccgt tactctcgct 5820

tctttattgt atattggtcg agtgacagca aaggtttggt cgcaatgtgc aaatggttat 5880

caaagaggcc tacttattct taacggtcta ggtttttttt attctagata tccaccagaa 5940

gatgacgagg gcaaaggcaa tacacctgcg cagaattgca tggtgagttt ctttccacgc 6000

tctatgttta ctactctcat ttccaacgcc atcaggtgta ttatcaccgc ctcggggaga 6060

aacaggaaaa agatactctt gtctagtaag ttcatgaggc atcaacacat ggccttgctt 6120

atgaatattc tgaaagtgag gatcccgagc atccattctg gctctgggca ctgcagctct 6180

cccctaggtt agtgtttccc agcactcctg atcactgtat tcacttagca aatatgtagt 6240

ggccgatatg cactcttgac tgcaagcgta cgcaaaactc atactatata cacatctcac 6300

tctaatgttt agctacacat agcgggatgc tagtcatact caactagcca aaattgctga 6360

tattgggacg agcgacattc aaaacggtat tcaatggttg actatccatg atcaatggca 6420

ggctagattt gttatgtacg tctaccttct cttctgcttc tcgacctcga ctaacattct 6480

agttaatact gatctcatta gaattggaga cgatgattca acaatttatt tcatgacgaa 6540

cctagaagct aaaaattacc tagtagcaac cttggacatt cggcattcag aagcgggggt 6600

caagacactg gtggctgaaa atcctgacgc ccttctcata tcggcaagca tcctctcgac 6660

cgataaactc gtcctcgtct accttcataa tgccaggcat gaaatccatg tacacgatct 6720

taacaccgga aaaccgatcc gccagatatt cgacaatttg atcgggcagt tttcgctgtc 6780

gggacggcgt gacgataacg atatgtttgt ctttcacagt ggtttcactt ctcccggcac 6840

catatatcgg tgagctcgtt gataagacat tgtggcaccc tcgccgacat tgtacaggtt 6900

ccgattgaat gaagatagta acaagggcac cctatttcgt gctgtacagg ttcctggcct 6960

caacctgagt gatttcacga cagaatcggt atgcatacgt gcttctcttc atcgctccta 7020

ttgctaagca ggatatgcag gtgttttatc catcaaagga tgggactccg taatcattct 7080

tgctgaatct cttaaacatc cgaagcctga taataaattt agtattcaca tgttcattac 7140

tcgattgaag gatacccccg tcgacggaac tgcccctgta tatatttatg gctacggcgg 7200

cttcgccctg gcaatgctcc cgaccttctc tgtctcgacg cttctgttct gcaagatcta 7260

ccgggcaatg tatgtggtac ccaacatacg gtaattaatt catcatcgtt tccctttcgg 7320

tctctgaatt ggcttatgct tcagtggtgg ttcagagttt ggagaatcat ggcaccggga 7380

ggtgagtagt gtttaatttt tgcgtaaaaa ataactttgt ctcaggcgcg ttctacaggg 7440

aatgttggac aagaaacaga acgtgttcga cgacttcaat gcagctacga aatggctcgt 7500

tgcgaacaaa tatgccaata aatataacgt tgccattcgc ggaggatcca acggaggtga 7560

gctgagctgt aactgttcga acctttgatc acagacatta agtgatgtgg tttttctaca 7620

ggagttttga caactgcgtg tgcgaatcaa gcgcctgagc tctaccgctg tgtcatcacc 7680

attggaggta tcatcgacat gctcagagtt tgtcaccata tccttcgttc tgattcctcc 7740

taattcctca ctcatccatg acattgcagt ttcccaagtt tactttcggc gctttatggc 7800

gttcggaata cggtgatgta ctaaaatgtc aattggtctg aaaaaatctg accctttata 7860

gcccgaggat ccggaggatt ttgacttcat ttacaagtga gctgaacagt cccgatacaa 7920

gattgcttcc ttacccaaat cttgacaggt attcccctta tcataacatt ccatcaggag 7980

atgtagtctt gccagctatg cttttcttta ctgctgcgta tgatgaccga gtttctcctc 8040

tgcactgtga gtgggccaaa atcttgaggt cttatagaac tctgatcggt ttaaagcatt 8100

caaacatgtt gctgccttac agtataactt ccccaacggt ccaaacccgg ttttgatgcg 8160

tattgatcta aacactggac atttcgccgg caaggtttga aattttttac tgtggtctcg 8220

cactttgcta attccatccc agagtacaca gaaaatgctc gaagaaaccg ccgacgagta 8280

caggtgcgac ttactctgtt gtaacctaca attgtgattg atctcaattt agttttatcg 8340

ggaaatccat gggactagtt atgtgcgctc agaacgaaca cgcgtcaaag caatggtcct 8400

gtgtcgtgac ctgaatcaaa ttgaaactat ttaacgcctc cctcgcccga agtcccaaat 8460

atggatccgt aaccgtttag gcgccgcacc tcacaaatag tttttcgtaa cgatttagaa 8520

aagtcctacg gtacaggatg ggctagtcag ttcttcactg aggctgtaga acgtataata 8580

gggataggtc agatatatga aatcagtgtc gacttttgtt acactactca actacataca 8640

acctttggag gtaaaacaat aacaattagc aactaggtgc tgatcgtcta cggaacaata 8700

atccagcagt ctggtttgga gcgggtgaaa aagtatacca cttatccgtg ggtcgtctac 8760

cgttatgttc cagcttgatg tcgtatgtca tcaagacgtg agcgagcatg gtcttcaaca 8820

gcgctgatac tagaaagcgg ccaggactga atacgttcac aaaatcatca aaatatgcgg 8880

atagttcttt cacatctctc accaagcatg ccttccatta ccaaacatca tgaaagtagg 8940

acccacggat accatttggt atcgaacagg gtcatcttgt tcttccctca gtcgagcaaa 9000

tctgaacccg tcaaaatcag ctggattgga atagtgcctc taggtatact ctttatattt 9060

tattccgagc agacgaaaat gaaaggctta cagcatcgtg gtgtatcgca tagccagggg 9120

caataataga gctccctgcg aagacagtcg tcccattgga gaacgtgaaa tccttcagaa 9180

ccttgcgggg catgctcgct gatggatcga atcatgaatc acacctggga gttcgcaaag 9240

tcaacgtacc ttttccacct gcccgtaggc gtagagactc cttgataaag ctatcgagct 9300

tatacatctt gccaatggaa tccttggata taccttcctc tttgacaatc gattctactt 9360

cagctcgtag ctccggaata tatactggaa atgtagccag gtcaaatagc gcttgtgtca 9420

aggcctgagg ttactcatga tataattcga agtttatttt taacaacatg gttcctcacc 9480

atagatgctg ggcgatacgc cgcaaacatg gtcgctaaca gtctgattgt gattgcctta 9540

atcgtagtcg cagataattt atcgctttcc ggcgttttct caatcagcca agtgagaaga 9600

tcattctggt tacactctgg taagcagata ccatacgata tttgagaact ttgctcactg 9660

attttccagc ccaatcggcg ccgaacaatt tttctttctc aaggcgttct tcgacaagag 9720

gagaaatgtg tttcattgca cgccgcagag ttttgggaat gtagacatca agaagtctgg 9780

agacaattct acaacatcat ttcaaaagag tatctagcaa aagcaggatt tagaagactc 9840

acggcttgag gaaaccaggt gtgaaaccta tgatcagagc agttttaaca agagcatctg 9900

agtatgcatc gttgatttca ttgtagtctg aattgttgcc ttgatgcata tgcggtaaac 9960

cacgagcgca atgatcatag cgagataaac aaacgtacaa agtgaaggac caacgaataa 10020

tctgttgacg actctgagga ccacaagttt tccagtaagc caagccggaa aagtggtcca 10080

gtctcttgac ttgttagatg aaaaatctcg aaggacaaga atgaatttct cactatcagt 10140

tggaggcaca agctctgaga aagctgcaat gacctcttcc ttgagctcag gaaaacgacc 10200

tccaagatcg cgtgttaggg agagatggat aaatcgaaag tgataagggt tctcgagaat 10260

gtgcggctcc atcgtgtagt cagtttgcat accctgaaaa attgcttgag atcatgaaca 10320

atagaagtat tgaggaacaa cgaaagacct ctttgataga gccatataaa gacaattgat 10380

cgtcgctcgc tttccggatg tcctcgatga gatcatgacc ggagacaaca attgtcatct 10440

ggaacaaagt aggaattgcg aacaactctc catagtgcta atggtagtaa tcgttagcta 10500

gtgaaacaat tagcaagtag tagcattcat cggcacacct gatcgtagcc ttcctgaata 10560

aagtcgcggg ttccgagtag aagctgaaaa aacccaatca cagttccaat ccctgttggc 10620

ccgacaaata ttatgttctt gtacttcaaa gagatgttat atgtctgggc atcaatgcat 10680

gggctcaaag cgtaaccata cctttgactt cctcaacttg ccatcagcga tgcagcgagt 10740

aacggtgagg aatactaacg ttataccaaa tatgaaccag gtagtcgagg atggcataat 10800

gaggggaaag ttgctcagtc gaaggattgg tgacaaattt gaatctccgc gtcaaataat 10860

atcattcata aaacaatcgg cggtgacata gacactcggt caataactgt gatgaggata 10920

agaatcatag gatcaagttg gtgaaaccag ggtgaaacga agccgtggtg gatatttact 10980

tgattgacat ctcgcttgga agatagctgt agcctgtggg tgaaatcaag tctaatcaag 11040

tagataatca ctgacaatga aagcgatttc tcagcgatac ttcggtcata agaggtcatt 11100

tatatttgac catccataat atccactatg gttcgcgccg ccgagttatg tagacagaaa 11160

gaatgcacga aaagaatttc acttttagta gttctttttt tttctggacg aaagagcttc 11220

ggacctcagg ctagcctcaa ccagcctcaa cctcagcgag ttctccggcc cgcctctcag 11280

caattgatgc aatcaaaagt ttccatgtat aggaatttcc ttccccaata tggataatct 11340

tttcccgatt gccatccgcc cactaatcct tttcatattc taacgatatt cttgatacat 11400

actcttctag aatcgcgcta cctggcggat ttccagaaag aatatcgaaa agatcagaaa 11460

tctccagata tccgatttga atcctgtcaa atttttatat ccttcttgcc acctggacta 11520

gtctggttct tggaaaatga caacgttgga gcaagagagc taagttaatc gttagtgacc 11580

acaagactca gctcccatgg gccctgcagc atatttagca gaatcaaatg tcaagctgcc 11640

ctgaatttta aagatctatg tacgcagtta gattttccct tgtggctcgc taaatacata 11700

ctgctaactg catgcgaatg atactctgca gccaatcaca tgagatgcag aacatctatt 11760

tgaacggggc ttatgccttt ggctttgctt ctcaccacca ccaccatacg actgagccca 11820

caccatgact gataccacga agattaaaac cgttcgaaag ttccacgacc tcctgaacga 11880

aggaaagctt gatgaggctc atgagctctt ggccgaagac ttgtctgcgc acaccactgc 11940

gcatatcgcc gcaggggcac ccccgatgag taaagcagag gttcttgaat ctcatcaagg 12000

cattcatcaa gagctggaag ctaaggttag tctctcagtc tcctctacac tgttgtaggg 12060

gcttcaactg agtatcatct agacaaccat caccgacgcc aaggaggtcg atggaaatgt 12120

tatcgtagtg gtgagtcata atgatcatat aaaacatcat gtaaaaattc taggtttaac 12180

gcgttctaaa tgattaggga aagtccactt tcaatcacga cactatcaag tatcattacc 12240

ttctcccatt cttattgcga ctatctttga tgccttttta gggaggttcc gttcaaggca 12300

acctatgaat tcatcgggaa tcagattggc aaagtccatt tcgagacgga tcctaaccac 12360

aaagttaaat ccttcagcct ctaggagctc tgcgggatct ttgaattgaa tctgtgggac 12420

cgtggatgaa tttcccggtg gtcaggaata catatgtagt ctatccgttg ttttgaagag 12480

cacgtatttg ttgtttgtat gatatggatg caagaaaagt tgaagttgtg tattcaaata 12540

acattgactc gtgaaaaaaa ggaggggact gcgaaagagc agtgctattg agtacggtac 12600

acgagtgaca agtagtacgc tgtacatgta gatatcgcgt gaaaagcaac ctcgcaattg 12660

tatatgagtg aaagtgagtg ctgtcagagc agatcacttg gctacaagca actttcaagc 12720

aagtccgatg aaagaattgt acaatacgac caatcggatt tgatgctaag tcctattctg 12780

tcctagaggc tattgttact acttccatac ctcctgccaa caacggacaa tctgggctcc 12840

tctgttgtac tttcccaact tgagctactc catattcttc tgcgttggag taattcccac 12900

tggcagggac ccaggcacta tcatcatagc tataggaaag gctcaggaga attccgatat 12960

ttgacagcaa ggatgttggc attaacgctc gctacactca tcctagcccc gttttgcaat 13020

tctcttctca accataatct tatggaatat tctacagata gatcatcagt gtattcattg 13080

cgtccgaaaa tcgcatttcc aacagacggt gaaatcttcc tgcaggatga gcgatacttc 13140

atttattgaa gtgcagagat atttcagata tataaagcct ttgaaaagta gccaactgtg 13200

attcagcatc acctacatag tcatcacaat ggagactcct accttaaaca aatccggctc 13260

cctcactatc gttggcactg gcatcgagag tattggtcaa atgacccttc agaccttgtc 13320

ttacatcgaa gccgccgaca aagtttttta ctgtgtcata gaccctgcca ccgaagcatt 13380

catcctcacc aagaacaagg actgtgtcga cctgtaccag tactacgata atggcaagtc 13440

caggatggat acctacaccc aaatgtctga ggtgagatgt ttcttgtcaa atgttttctt 13500

tgtgttctgt atagagctga catcttcaaa ggttatgctc agggaggtcc gcaagggcct 13560

tgatgttgta ggcgtcttct acggacaccc aggtgtgttc gttaaccctt cgcttcgagc 13620

gctcgctatc gccaaaagtg aggggttcaa ggctaggatg ctgccgggcg tatctgcgga 13680

ggactgcctt tatgcagact tgtgcatcga tccttcgaac cccggatgtc tgacctacga 13740

agcatctgac ttcctcattc gagagaggcc aacgaacatt tacagccatt ttatcctttt 13800

ccaagttgga tgtgtcggta ttgctgactt caacttcact ggatttgaag taagtatcat 13860

caatctccat gaggtataca tttaataaac tctttgttct agaactcgaa attcggaatt 13920

cttgttgacc gcctcgagaa ggagtatggc gcagaacatc ctgtcgtgca ctacatcgcc 13980

gctatgctcc ctcacgagga tcccgttacc gatcagtgga ccattggaca actccgtgag 14040

ccggagttct acaaacgcgt cggcggtgtt tccactttct acatcccgcc aaaggagcga 14100

aaggagatta acgttgatat cattcgtgaa ctcaagtttc tccctgaggg gaaagttccg 14160

gatacgcgta ctcagattta cccacccaac caatgggaac ccgaggtacc gactgttcct 14220

gcgtatggat caaatgagca cgccgctatc gctcagttgg acactcacac tcctcccgag 14280

caatatcaac ctctcgctac ttccaaagcc atgactgatg tgatgaccaa gttggcgttg 14340

gatcccaagg cgctcgcaga atacaaggct gaccaccgag ccttcgctca gtccgttccg 14400

gatttgacgg cgaatgagag gaccgctttg gagattggag actcctgggc gttccgctgc 14460

gctatgaagg aaatgcctat ttcactccta gacaacgcca agcagtcaat ggaagaagcc 14520

tccgaacaag gcttcccgtg gatcatcgtc gttggtgtcg ttggtgtcgt tggttctgtt 14580

gttagtagcg cctgattatt ctgattgcct ctcaaattca aaacactgta acaactacga 14640

taagtattct catatgtatg taaagaaata ttgtcagtaa atatgtcact cacgaggaat 14700

gcctgagcct cgaaatatca catatctaac attgcgtgta ttattgtttc catacaatca 14760

acttccttct acacggacct aatgaacaat tagtagtcag atgcatctcg cctcctctcg 14820

cgcggttgct caagctggcc cctgcttgcc cctcctacta tgtagcatga taaggcatca 14880

agatccgatg tgaacgtaaa aacctatcag aatcaggaat gaatataaat ccctgatatt 14940

caaaggttcg acgaggaacc tcagggaact tcaaagcctg aggtttggtc tgggttcggt 15000

tacgtaaaac gacgtcacga atctcgaaat tttcaaagct tcatgttgat gatctgtgag 15060

gagagcattc ctgtgatttc gacacgacag caaggatttg aaggtgtaca tgggctgaac 15120

agacgcttta tattatcaca gcaaagtaca tagcaatcat caaagactca ccttaatcta 15180

cactgatcca atcaccgcct agttagttga caggtaacac gattcgctag gatgacaatg 15240

atatgcaact caatcatcac aagacatgcc tttgccttgc atttcctttt tcttgagcgg 15300

aaatgaaagt ttggaaagag atatataaac tgagctaaac tgtcacgtac gtgggaattt 15360

caaataatat ggcgatattt tcttccgttt tcctgttcct cctagcggtg ggacttgctc 15420

tgctaacaat tcttacgagg cgcaaaatca aggaaagcta tatcggcttc cgtgctatag 15480

cttacagcta tgacttgatg tcatctagct tcagacccgg ccaaaacacg tatcatttca 15540

ctattcttgg agtatgtact agcagtatta ttccatctta ccgctgtgat gtttaccact 15600

gaagtagaat cctgtggttg gattgagagg tcaagaagcg aggaaagtgt tcttcaacga 15660

aaggaacttc agtactagtg aaggatacaa ggtgttcagc aaagcagctc ctgtatgcga 15720

actgcaaatc tccttatatg ccattgttga agctttttaa caggttccag aactagctaa 15780

aacgttactt tctgcagagg aaggccatgg acttcacttc aagaagcaac ttgaaattct 15840

cgtgaacaag aatcggttag ctgacagtaa gcagcaaata gctacgtgta gtaggctcat 15900

atttaaatac acggcagtta tcgcaactat gctgggggat atgcagcggc tcacggctac 15960

ctggaataat aaagggtcga tggatccttt tgacaaaata aacgacgtac gtgttgccga 16020

ttttactaga cctcgattaa aacttcgtct acatagatca cctttcatgt taccgcgcat 16080

atgatcacta gcaacgactt cgtttcagat ccagtgaatt tctctcgatt tcaaacccat 16140

ttcttcaccc aactcaagaa taacactccc gcaatgattt tcttaccgtg gttctatcgt 16200

caatcacaac gagaaaaaga aatagctata acttccctct tcacgacctt aacgagctat 16260

atcgaagctc gagaaaagga caaaatttcg acttctgaac ccatcgatct gttacttgac 16320

cacgggatga attcacagga gatcatccag ttcattctcg gattcctgtt tggaggtgtt 16380

tacaacgcaa ctaaagcaag tacgcgtcca ataccttttc aagttttatg tgattttacc 16440

gcgttgtata catagtctcc tggatactta tctatatctc gatgcattcc caatggaaaa 16500

tggccatccg ttccgaggta gaaaatgtcc tctccaaaca ttctttcgat atctccaagc 16560

cgctatacag cagactctct gaagtaccgc tcgaggcctg ggaggaatcg ctgcctacgc 16620

tcgacctcgt catttccgag acatttcgcc tggtcatgaa tcaaaccgtc ttacggcgga 16680

atataggcga agatgtcgaa atacctggcg gacaggccca tgttccaagt ggagcattcg 16740

tagcatatag taatgccgat gctcatctgg agcccgaaat atacgacgat ccttggtcat 16800

tcaagcctga tcgttttgaa catggaaaac agaaggtact ctcaaacgac ttcttgggat 16860

ggggcgctgg taagcaatgt tttcatatgt tgatgccaat gaatttgcaa accatccaat 16920

accagggaga caccgctgtg taggattggg gatcgcaaaa ctgacgatca agacgattgt 16980

ctgtttattc gtgatgacct acaattacga tataactcgg acgaagcgca agtctcaccc 17040

gacaattccg attcctgatc ataacaaggt aaatgaggtg agtgtcttga agaagtgtac 17100

ggcctacaga ttcattaaaa tcttaatacg caggcacgcc caaagggtga agtgattact 17160

ctcaattacc aacgtattga acaatctgtt tcaatataag ttggatgttg tgactcgtac 17220

tgtaccacac gaaacctgag cacagagaaa ggacaggaag atgctcttta tgtcaacact 17280

tttgagtgtt acaaatgtgt cctcttgagc ctgctgccgg aataaaacca gggtactttg 17340

gcgcggagac catgtctttt gacttcggtg tatatttgaa ttgtaatatc actgccagac 17400

aacgagaaaa tactgcagcc aacaacctga atccggggca tatcgaatga gatcaatttc 17460

gaaaatttca gagcactata gttccatatg gtacatacaa caagatctct gaggtcgtgg 17520

tttgagccat ctatattttc acaacttctt cgagttcacg atctgtacaa attcgggttt 17580

cagggaagca cctcccacaa ggaaaccatc gacgtcgggt agagtagcta gaacatgggt 17640

tgaggtcgat cagagacaga tagatattcg gcaggaaaaa tggttcaaaa caaaaagctc 17700

acaaagttct ttgcaattcc cagcattgac gctacctccg tagatgatcc tggtcgattc 17760

agcgacacct gacgagacag ctttggatag atacgaccgg atatctttgt gagcctcctg 17820

cgcctgctgt ggagtagcaa ctttgccagt tcctatggcc cacacgggtt cgtaggcaac 17880

gacgatttta cttgtagata agatgggaag ggagataaga cgctcaacgt atgaaagatc 17940

tacacaaaat ctcacctcca atcgctctcc tttaatacat ctacgacagc tttgagttgt 18000

tcctcacaga cttttgcggt ctctccggcc tcgcgttcgt tgagagtctc gccgatacag 18060

agaataatgt tgagtccctt atcgagagca gcgcgagtct tttgcgcaac gacttcggaa 18120

gattcatgga agagggttcg acgctccgag tgtccttgag gttaacaaga aaaacatatg 18180

agtattcggc tactgtcatc atacatagtt aacgcaccaa ggataacgta gggtatttca 18240

gaatctacaa gttgtgcagg actgcatatg gcatctgagt tcgaagtttg caatgtaaat 18300

tcggaatcgc gaacctgatc tctccggtga aagcaccaga gtctttgaaa tagcaattct 18360

gagcagctac tttgatgtcc ttccggatgg acccttttac agggataaga tatagagctg 18420

gtggagatat gacgacctct atatatcaag aagaacacac gggcaaagaa taaggtcaga 18480

tgtttaatga gagtgtgagt gagtaagacg tgccagtgtt aggatcaagc tctgcttcgt 18540

tcaaaagctt caccagagct actttgtcct tccggctagc cggattcatc ttgaaattgc 18600

ctccaacaaa gaattggcgc gccatgtagt tgctcggata ggataatgag tgaagtgcaa 18660

gctgatcgat tgctggtggc tcaaagttta tttgttctca cgatttgctt cgatcagttc 18720

tgttccgatt acagtatcgt ccttcttcgc cgctatagtc cttgcacgta ttctcaatct 18780

ggatgttatg caatcgccac tgagctgaat gtgcgtcgtt agtaccgtat gcttcaaacg 18840

ttgcggactt gatgctactc gatgtgcttt aacaggcttt aactcatctt tcttcgtgca 18900

ttaatatttc aggttaactt tgggatggac cttgatagac ccagtttcac ggaacgatct 18960

cgaagtaatg ggccaacggc attgacagaa aatggatacg acccgccggt taggttgacg 19020

gttatcagtt ccgtttcagt cattcttcct gacacattca tattgaacgc gtgcgtgtta 19080

ttttgcctta tttgaaccaa ggtcatatta taactaccta gctgactatt ggttcctcga 19140

agaagtggat tgatctttca tgcacactga cgacacctaa ctcatcgttt caactgctta 19200

aagcattgcg caacggtgat cgtctctccc tcgataacat gaaactttac aatcctttat 19260

acccacctcc cgctccacct gcatttggca gagatacagt cctaccagaa ccaaaggcat 19320

cgtgtgtcat tgtccatctt gatgtcgtct ttgcctccct cacgcttgga aatttcacag 19380

actactctcc cgcctcgtat ttcagtggtt gtcgccgttt ctagctgtag gattctctcg 19440

gcctctggag aaggacggtg agctatcgac acgaaatctg ttcattgctt acctccttgt 19500

cattcgattc agatttatgg caactcccct ctgcacgtct caccgcgaat ataaccgata 19560

gaatcgagac gaatttcttt caacgatgcc cccctgacca gaggcctcgt catttacgtc 19620

aaaagctccg tcaattttgg gaagctgaag tgacggacgc gattccagag caggacaatg 19680

acaacgatag cacgtacgaa ggctcgtcag atacgcagaa gaccgacgag aagaagagct 19740

tcaacaagaa gaagaaaaac atagaaccta aatatgattc ctcattatta caagccatat 19800

acataacttt taaagccgaa tggtggatct ctggaatgct caagttaatt tcaggttacc 19860

ccgatttcct tcttctctgc tcgaggtcgc aaatgctcac tcctaactct atctgctcct 19920

ttagatacac tcaagaccac gacccctctc gtcaataaag ttattctcac ttggcttaca 19980

gactcgttca tattctacaa ggctacgcca tcagaaagat ctgagcttgg attgacttcg 20040

cctcgtggta tcggatatgg gattggactt gcattcgctt tgttcgttat gcaaggtatg 20100

tacttgttta tttctcagaa atttactcac atctgcctta gaaagcgcta gtttggtgag 20160

acattgacaa cccgttataa ttttacccca tctaaatttg ctctagatga cgaatcatta 20220

catgcagagt atgtcaatat cggtgcagtt gagcaagaac tttactaacc cagcccctgc 20280

cttgtaaaca gtaagcatgg ccaacggatt agccatcaga acgggcgtag gttgtttcac 20340

aacagtacac attccttcaa tttgaacatt tttcgtagat catagggaac atattccgca 20400

aatccttgcg tctttctggg cgtgcaagaa accagcatag cgttggacag gtgcacattc 20460

atagaattcc cgtattttac accgagctca aacagataac agataaccac aatgatctcc 20520

actgatgcta cgaggttgga cacattctcc gccaatgtcc acaagtgtgt tatacttgtc 20580

cttttgtatg cttactcctg acatgtaatc tatgaagtat ttgggtatct ccaatccaga 20640

tagccattgg tatcgggctt ctcattgcaa acgttagtat ccttacctgc agccgcattt 20700

atgacgaaga tttgatattg tatctacttt tatggcacag ctgggatatt ctgcacttgt 20760

gggattgggt gtaagtcata gtatgcttat cccgtgttgc atgcaatcgc gtctaatttt 20820

ctggatctag gtacttctct tgggactccc tttgcaattc ctttgcctga ggataatgtt 20880

catgaacaga agaaagggag tgaaggtcac agatagtaga gtccggctta ccactgaggt 20940

atggataata atttcatgca cttggatacc tatgaaccaa gttttgcttc cacaggttct 21000

gcaaggtatc cgcctcatca aagcatatgc ttgggaggga ttctatgttt cccaaatcta 21060

caacttcagg aagcaggaag tggcacggat tcggactgct tcgtaagtaa attatctctg 21120

catgagtttg tcactgttct tgtgttaact tcaccatcca ggatagcccg cgcagttctc 21180

attgccctgg tcacgtttat cccagttctt gcttctgtcc tctcctttgt gagcttccgt 21240

tgtgtataga accagaacta cgtattgatc aacattttta gatcacttat gcattaagcg 21300

gccatgatct caatgttgcg attattttca gctctttaca attcttcaac gtcagttaac 21360

atccgtctgt agtgtagctg tgacactgac cctgacctcc gacctgtttt cgtagattat 21420

tcgtgctcct ctcatctttt tccctatcgt cctctcttca ttgtccgact tcctcgtcgc 21480

catgggtcgg attggcaaat tccttaaagc agaagagctt gcagaatctt acaccatcga 21540

cttcgaacgt aaacatgctg tggaagtcga tggcgacttc agttgggaat ctgcaggtaa 21600

aatagtagaa acaaagtttg aaaagggtgc aaagaacgac catgtcccaa actccggtgt 21660

gaaatccaag gaagatggaa agcctgcaaa ggcggtctca aaaagcaccc aagacgaaac 21720

tctacccacc acttttcaag atatcaaaac gacgcaagac attgatagtg atcccctgaa 21780

ggaagatgaa cccttcgtat taaggaacct caagttcaac gtcccgaaag gttcttttgt 21840

cgccatagtg ggcactgtgg gttcagggaa ggtgagaccc aacccttata atcccatccc 21900

gccacgtcta atcgtgccct ttctgtgcag agttcaattc tgaacgcttt gataggggaa 21960

atgcggcgaa ctcgaggaga agtagttttc ggtggaactg tgtcctatgt cccacagact 22020

ccatggataa ggaatgccac tctgaaagag aacgtgctct tcggcatgga agaagatgat 22080

aaaaagtaag cattgttcat tcttcctgat ggcactgaca tgacatgaca actccgtagg 22140

ttcaaagaga tcatgcgcgc ttgtaatttg gagcacgatt tagaactact accgcacggc 22200

gagtatactg aaattggcga aaagggtatc aatttgagtg gtgcgagcat taaccgaacg 22260

actaaaaact actgctcaat tcatgattct aggcgggcag aaggtagcaa ccatcccatt 22320

tcgtatattc gaggttgaat ttgtctcact ctctcgtttc ataggcccga gtttcgttag 22380

cgagggcggc atactcagat tcagatatcg ttcttctgga tgacccatta tccgctgttg 22440

actcatatgt gggcaaatct atattgacgg attgcttcct ggacggtcca ctggcagaca 22500

aaacgcgaat tctggtcaca catgctttgc acgtgcttga caaaacagat tacgtttacg 22560

tggttgacca gggagaaatc aaagagcagg ggacattcca ggtgtgtaaa attcatacta 22620

gtgtgttgca gctgtcgact catgtctcca gacgcttatg tctgccaatg gccttttcac 22680

gcggatcatg gatgaatatg gaagcttaaa tcacgagaac gaaagtaaca gcgagaagcc 22740

aaggacggag gatgaagtcg taaatcctat acccgacaac aaggctgcaa atgctgctct 22800

tatgcagaca gaagaacgta acattggcgc tatcgggtat caaacgtatg cgaaatacct 22860

aaaattcgcc ggagggattt ggtgggcgcc tttcattctt ctcctgttaa ctttaacaca 22920

aggagcgcaa ggtgcgtcag ttcaactttt gatcgagagt tcagctgaaa ttccttcgaa 22980

ctttagtcgc aaataatctg ttcttgggct tctggactgc ggaaagcatt ccaggttttg 23040

gccaagctca gtatatgggt gtttatgcag cgcttggtac gggaggcctt gagcattgat 23100

atatatgggt taaccgcact ctctaggttt tgctcaagcc ctgttcacgt ttctgttgtc 23160

cttttcgttt gcgtgagttt atcggccaat tatttgctgg atcgagatcg atgaccagat 23220

gtacgcttgt agtattgctg gccttgtagc aagcctcaat ctcttccaaa cagcactccg 23280

tcatgtttta cgatctccaa tttcattctt tgacactacc cccatgggtg agtaagcgtt 23340

cattcgcagt gtccttcaaa cttagaaatt tttcattagg tcgaattctc tcccgtttat 23400

cgaaagatca agatacttta gactccaccc ttacgatgac cttgatgcaa tttctgaaca 23460

cttttagttc tgtcttgggc accgttgcac tcgttttcta cacttttcct cttttaggga 23520

tcatctttgc acccatgaca gtgttctact atttcatctc catttattac cgacggtcat 23580

ctgtcgaaac gaaacgtttg gattcgttga tgcgttcggc actatatagt tcttactcag 23640

gtaatctgca ctcgtctctg attttctgac atcagttgac agggatactc cattgtagaa 23700

actctcactg gtctgtccac tattcgtgca tatcgcgaac aggcaagtgt ctccccctaa 23760

cgctatgtcc ctcaatctaa acagatgcca caggcgcgat cggttttaaa tgcgcaaaat 23820

ggcctcgatt tggagaatcg ggcgtattat atgactatat ccatccagcg atggctatct 23880

ataagattag attttctggg aaatattttg attttgggaa ttgccctgtt tgccgctggt 23940

ttcagagaaa cagtgaatcc ttcgaagatt ggggtcgttc tttcatacac gcttagcagt 24000

aagtcattaa tttcactcta tcagacaagc tcctgcctga acttgttttt gatgcagtaa 24060

ctcagatatt ctgtatgttg catcttgtcg tgttctatct cctttcgatc cattgagcct 24120

gctttcatag cggaaatggt cagccaatac gctctgagcg agcaggtaat attcacccca 24180

gttccagctt tgaaccaccc taacacccaa gtagaatatg aatgccgtgg aacgtatctt 24240

ggtatatgca gaattaccaa gcgagggtga cccagtgacg ccgcatgatc cgcctccatc 24300

atggcctcat gcgggacgcg tcgaattcag caatgttcag ctggcctatc gaaaaggtct 24360

tcccctcgtg ttgaatggcg tttctttcaa tgtgcagccc ggagagaagg tgtgttggcg 24420

aaatacttcc ccacgatctc aatattaatt ctgcgtatag attggcatag tcggcagaac 24480

tggggctggt acgatgacag tgttttggaa ataccgacat cactcacatg gacaataggt 24540

aaaagttctc ttatacaggc gctgctccgg tacgatgcct tatccgatct caatccagtg 24600

cgccactaac atcatgcatt agcatggttg aacttgaggg agggaaaatt gagatcgatg 24660

gttttaatac ccgagaaatt ggcttggacg ttctaagagg gaagttagct ctagtgcctc 24720

aagatagtgt tctttttctt ggaacactgc gcgagaattt gtaagggtct attacatgac 24780

atcctatgtt cctcacggtt ttaatatttc gatagggacc caatgggcac tcgcacggat 24840

gcggagctga tctccgcatt gcaacgagct tggttgttgc cgcgcgatgg aacgaaggat 24900

cccgtggctg aagcaaaatt cagtttggac gccaccgttg gcgacgaagg tgagaatccg 24960

ctggaattgg ttctgcattg aggacctcta aatacgtccg gaaggctcca attttagtgc 25020

aggagaaaaa caactcttag ctctatgtag agctcttgtc aagaaaagtc gaattatcat 25080

cctcgtcagt aaaagttttc agcacataca tctccatact aacactctcg atcatgtaaa 25140

ggatgaagcc acaagcagtg tggatgtgga aacagatgga aaattacaga gaactattca 25200

gacagagttt tcgacctcaa gtcttctctg tatagctcat cgcttgaata ccatcggtaa 25260

acccctactt ccaatttcct accgcgtata tacctatctg acggattcat gtagcatact 25320

atgatcgtat aatcgtgatg gatgctggac aagtcgccga attcgatacg gttctaaact 25380

tgttcgaccg tgaagactct attttccgat ccctctgtaa tgaagccaac ctacaacgtt 25440

ctgatattct acgcatacga gctagttatg gagatgtagc cccgatagag atgcccgcat 25500

aactgaaagg ccaagtaaat caataaatag agatatttta aacttgtaga ccttaaaaac 25560

gtagacaaac ggatattact tcttgaggcc ccactatttt cagcttctac atctatatca 25620

tggaatctct taattcccgc tacctttcca gaggtgaaat agccttatag ggccaacttt 25680

tcgtttcttt gacctttttt aatcaatatt taattgtaca atttttacaa gtctctgtag 25740

tcgacaatat atctatatac tgtcttccta ttctcgcagg agaattttac ttacaaatgg 25800

tatcatgggc cctggtagca ggccctgccg ctgtctgaaa atttatgtcc agcattttgc 25860

agggcaaaga aatctattac ccgaatatac gcaaggttcc 25900